# EUROPEAN PATENT APPLICATION

(11) **EP 1 912 130 A1**
(43) Date of publication of application: **16.04.2008**
(21) Application number: 06756749.5
(22) Date of filing: 30.05.2006
(51) Int. Cl.: G06F 19/00, G06F 17/30

(54) **PATHWAY DISPLAY METHOD, INFORMATION PROCESSING DEVICE, AND PATHWAY DISPLAY PROGRAM**

(30) Priority: 22.07.2005 JP 2005212824; 14.04.2006 JP 2006111916
(71) Applicant: Kazusa DNA Research Institute Foundation, Kisarazu-shi, Chiba 292-0818 (JP); NS Solutions Corporation, Tokyo 104-8280 (JP)
(72) Inventor: KAWAI, Makoto KAZUSA DNA RESEARCH INSTITUTE FOUNDATION, Chiba, 2920818 (JP); KOGA, Hisashi KAZUSA DNA RESEARCH INSTITUTE FOUNDATION, Chiba, 2920818 (JP); MURAKAMI, Masatoshi KAZUSA DNA RESEARCH INSTITUTE FOUNDATION, Chiba, 2920818 (JP)
(74) Representative: Vossius & Partner
(86) International application number: PCT/JP2006/310801
(87) International publication number: WO 2007/010677

(57) **Abstract**

A pathway display method in an information processing device processing Information includes a pathway information acquiring step (S10), an expression information identifying step (S11), an expression information acquiring step (S12), and a dynamic image display step (S13).

## Description

### Technical Field

The present invention relates to a pathway display method, an information processing device, and a pathway display program product.

### Background Art

A gene is a factor determining a hereditary character of a living organism and is considered as a unit of genetic information, and this genetic information is actually DNA. DNA is replicated, so that genes are inherited to a next generation. The genetic information can exhibit its function only after it is made into an amino-acid sequence of a protein through processes of transcription and translation, and these processes are called expression.

As one of means to represent a relation between genes and between proteins thus forming a living organism, used is a pathway visually showing the relation between the proteins, with, for example, nodes representing proteins and the like and controls representing interaction between the nodes. For example, in a process where proteins existing in large numbers in a cell cause one phenomenon while mutually related to each other (for example, cell adhesion and cell division, neurotransmitter release, and so on), specific proteins among proteins which exist in the number of about thirty thousands in a human being work at a specific phase to form one pathway. This pathway is utilized for analyzing the mechanism and the like of a disease.

Examples of a method of displaying this pathway are a method described in a patent document 1 and so on. The method described in the patent document 1 automatically plots graphs of a complicated pathway and medium-scale/large-scale pathways.

However, the above method described in the patent document 1 has a problem that it is difficult for a user to easily get a visual idea of processes of the expression of proteins, amino acids, and the like that occurs in time series since the method displays a pathway at each time section separately. Similarly, this method has another problem that it is difficult for a user to easily get a visual idea of processes of the generation of metabolites that occurs in time series.

Patent document 1: Japanese Patent Application Laid-open No. 2003-167882

### Summary of the Invention

The present invention was made in view of the above-described problems, and it is an object of the present invention to display processes of the expression of proteins, amino acids, and the like that occurs in time series, by dynamic images in the same time series or different time series. It is another object of the present invention to display processes of the generation of metabolites that occurs in time series, by dynamic images in the same or different time series.

To solve the aforesaid problems, the present invention is a pathway display method in an information processing device processing information to display a pathway including: a plurality of nodes each being a symbol representing one of a protein, an amino acid, a gene, another substance associated with a gene, and a group of proteins, amino acids, genes, or other substances associated with genes; and a control representing a relation between the nodes, the method including: a pathway information acquiring step in which the information processing device acquires, from a memory, pathway information regarding the pathway; an expression information identifying step in which the information processing device identifies expression information corresponding to the pathway information acquired in the pathway information acquiring step, based on association information stored in a memory and associating the pathway information and the expression information with each other, the expression information being information regarding expression of the protein, the amino acid, the gene, the other substance associated with the gene, or the group of the proteins, the amino acids, the genes, or the other substances associated with the genes that occurs in time series; an expression information acquiring step in which the information processing device acquires, from a memory, the expression information identified in the expression information identifying step; and a dynamic image display step in which the information processing device displays the pathway on a screen by using the pathway information and the expression information and displays a dynamic image by changing the display of the pathway in time series.

According to the present invention, processes of the expression of proteins, amino acids, and the like that occurs in time series can be displayed by dynamic images in the same or different time series since it is a pathway display method in an information processing device processing information to display a pathway including: a plurality of nodes each being a symbol representing one of a protein, an amino acid, a gene, another substance associated with a gene, and a group of proteins, amino acids, genes, or other substances associated with genes; and a control representing a relation between the nodes, the method including: a pathway information acquiring step in which the information processing device acquires, from a memory, pathway information regarding the pathway; an expression information identifying step in which the information processing device identifies expression information corresponding to the pathway information acquired in the pathway information acquiring step, based on association information stored in a memory and associating the pathway information and the expression information with each other, the expression information being information regarding the expression of the protein, the amino acid, the gene, the other substance associated with the gene, or the group of the proteins, the amino acids, the genes, or the other substances associated with the genes that occurs in time series; an expression information acquiring step in which the information processing device acquires, from a memory, the expression information identified in the expression information identifying step; and a dynamic image display step in which the information processing device displays the pathway on a screen by using the pathway information and the expression information and displays a dynamic image by changing the display of the pathway in time series. This enables a user to, for example, easily get a visual idea of the expression processes of amino acids and the like. Here, the memory corresponds to, for example, a later-described HD 19, a HD of another device connected to the information processing device via a network, or the like. The memory storing the pathway information, the memory storing the association information, and the memory storing the expression information are not necessarily the same memory. Further, a gene corresponds to, for example, DNA, mRNA, and the like to be described later. Another substance associated with a gene corresponds to, for example, a chromosome or the like. A group of proteins, amino acids, genes, or other substances associated with genes corresponds to, for example, a protein complex, a later-described ribosome, or the like. Further, a dynamic image corresponds to, for example, an animation, that is, an image displayed by changing a still image in time series.

Further, the present invention is a pathway display method in an information processing device processing information to display a pathway including: nodes each representing a metabolite; and a control representing a relation between the nodes, the method including: a pathway information acquiring step in which the information processing device acquires, from a memory, pathway information regarding the pathway; a generation information identifying step in which the information processing device identifies generation information corresponding to the pathway information acquired in the pathway information acquiring step, based on association information stored in a memory and associating the pathway information and the generation information with each other, the generation information being information regarding generation of the metabolite that occurs in time series; a generation information acquiring step in which the information processing device acquires, from a memory, the generation information identified in the generation information identifying step; and a dynamic image display step in which the information processing device displays the pathway on a screen by using the pathway information and the generation information and displays a dynamic image by changing the display of the pathway in time series.

According to the present invention, processes of the generation of metabolites that occurs in time series can be displayed by dynamic images in the same or different time series since it is a pathway display method in an information processing device processing information to display a pathway including: nodes each representing a metabolite; and a control representing a relation between the nodes, the method including: a pathway information acquiring step in which the information processing device acquires, from a memory, pathway information regarding the pathway; a generation information identifying step in which the information processing device identifies generation information corresponding to the pathway information acquired in the pathway information acquiring step, based on association information stored in a memory and associating the pathway information and the generation information with each other, the generation information being information regarding generation of the metabolite that occurs in time series; a generation information acquiring step in which the information processing device acquires, from a memory, the generation information identified in the generation information identifying step; and a dynamic image display step in which the information processing device displays the pathway on a screen by using the pathway information and the generation information and displays a dynamic image by changing the display of the pathway in time series. This enables a user, for example, to easily get a visual idea of the generation processes of metabolites. Here, the memory corresponds to, for example, the later-described HD 19, a HD of another device connected to the information processing device via a network, or the like. The memory storing the pathway information, the memory storing the association information, and the memory storing the generation information are not necessarily the same memory.

In order to solve the aforesaid problems, the present invention may be an information processing device and a pathway display program product.

According to one of the present inventions, processes of the expression of proteins, amino acids, and the like that occurs in time series can be displayed by dynamic images in the same or different time series. Further, according to another of the present inventions, processes of the generation of metabolites that occurs in time series can be displayed by dynamic images in the same or different time series.

### Brief Description of the Drawings

[Fig. 1] Fig. 1 is a conceptual view of a central dogma;
[Fig. 2] Fig. 2 is a chart showing expression steps of a protein;
[Fig. 3] Fig. 3 is a hardware block diagram of an example of an information processing device;
[Fig. 4] Fig. 4 is a functional block diagram of the example of the information processing device (No. 1) ;
[Fig. 5] Fig. 5 is a chart showing an example of node position information;
[Fig. 6] Fig. 6 is a chart showing an example of control attribute information;
[Fig. 7] Fig. 7 is a chart showing an example of association information;
[Fig. 8] Fig. 8 is a chart showing an example of expression data (No. 1);
[Fig. 9] Fig. 9 is a flowchart showing an example of pathway display processing;
[Fig. 10] Fig. 10 is a flowchart showing an example of dynamic image display processing (No. 1);
[Fig. 11] Fig. 11 is a flowchart showing an example of display processing of nodes and so on using expression values;
[Fig. 12] Fig. 12 is a view showing a pathway display screen (No. 1);
[Fig. 13] Fig. 13 is a view showing a pathway display screen (No. 2);
[Fig. 14] Fig. 14 is a view showing a pathway display screen (No. 3);
[Fig. 15] Fig. 15 is a view showing a pathway display screen (No. 4);
[Fig. 16] Fig. 16 is a view showing a pathway display screen (No. 5);
[Fig. 17] Fig. 17 is a chart showing examples of a display pattern;
[Fig. 18] Fig. 18 is a functional block diagram of an example of the information processing device (No. 2);
[Fig. 19] Fig. 19 is a flowchart showing an example of the pathway display processing (No. 2);
[Fig. 20] Fig. 20 is a view showing a pathway display screen (No. 6);
[Fig. 21] Fig. 21 is a chart showing an example of the expression data (No. 2);
[Fig. 22] Fig. 22 is a flowchart showing an example of expression information identification processing;
[Fig. 23] Fig. 23 is a flowchart showing an example of expression information acquisition processing;
[Fig. 24] Fig. 24 is a functional block diagram of an example of the information processing device (No. 3);
[Fig. 25] Fig. 25 is a view showing a pathway display screen (No. 6);
[Fig. 26] Fig. 26 is a view showing a pathway display screen (No. 7);
[Fig. 27] Fig. 27 is a flowchart showing an example of dynamic image display processing;
[Fig. 28] Fig. 28 is a flowchart showing an example of the display processing of nodes and so on (No. 2);
[Fig. 29] Fig. 29 is a flowchart showing an example of the display processing of nodes and so on (No. 3):
[Fig. 30] Fig. 30 is a functional block diagram of an example of the information processing device (No. 4);
[Fig. 31] Fig. 31 is a flowchart showing an example of pathway supply processing;
[Fig. 32] Fig. 32 is a chart showing an example of the forms of a control;
[Fig. 33] Fig. 33 is a view showing a pathway display screen (No. 7); and
[Fig. 34] Fig. 34 is a view showing a pathway display screen (No. 8).

### Detailed Description of the Preferred Embodiments

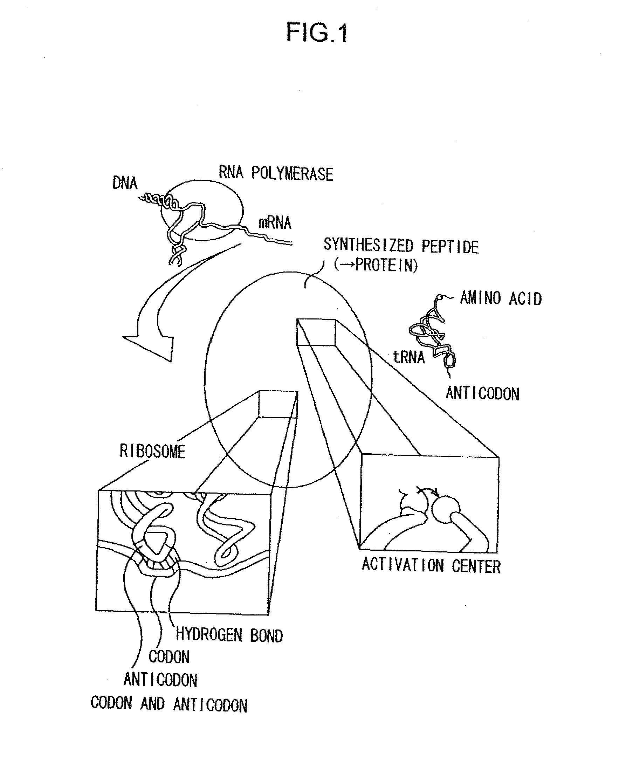

Hereinafter, embodiments of the present invention will be described based on the drawings. A concept of a central dogma premised in the description of the embodiments of the present invention will be described by using Fig. 1. Fig. 1 is a conceptual view of the central dogma.

DNA carries a design drawing of a protein playing an important role in phenomena of life, but a protein cannot be synthesized directly from DNA. Information carried on DNA is once transcribed to messenger RNA (mRNA) and undergoes a translation process, so that a protein as a chain of many amino acids is synthesized (central dogma). A ribosome and transfer RNA (tRNA) play an important role in this transcription. The ribosome takes in the mRNA, and tRNA as a carrier of the amino acids recognizes a codon in the mRNA. Then, adjacent amino acids are linked together by peptide bond by enzymatic activity of the ribosome and are made into a peptide and further into a protein.

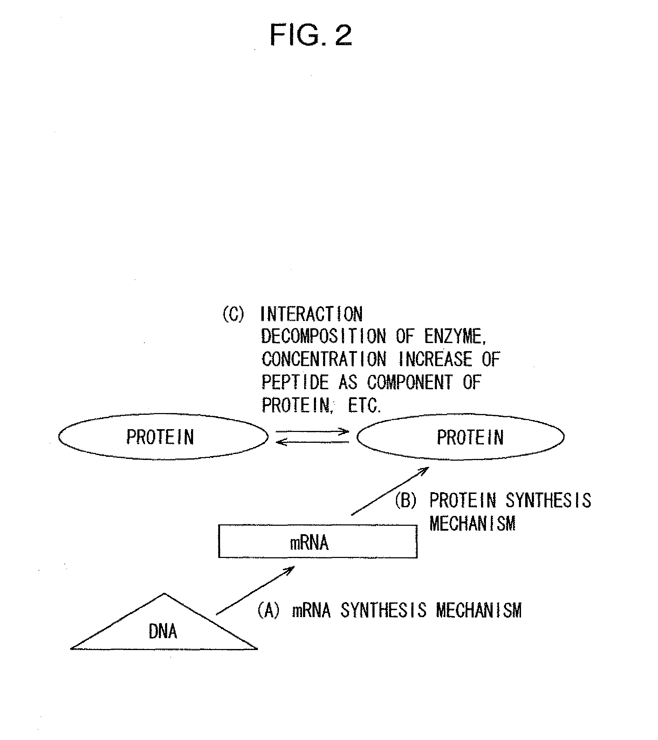

Here, expression steps of a protein are shown in Fig. 2. Fig. 2 is a chart showing the expression steps of a protein. Abnormality of an organism mechanism possibly occurs in (A) and/or (B) during the course of the expression of each protein. Further, some mRNA and protein have functions of accelerating or inhibiting the expression of other molecules, and therefore, abnormality may possibly occur in (C) inter-molecule (inter-protein) interaction.

### (Embodiment 1)

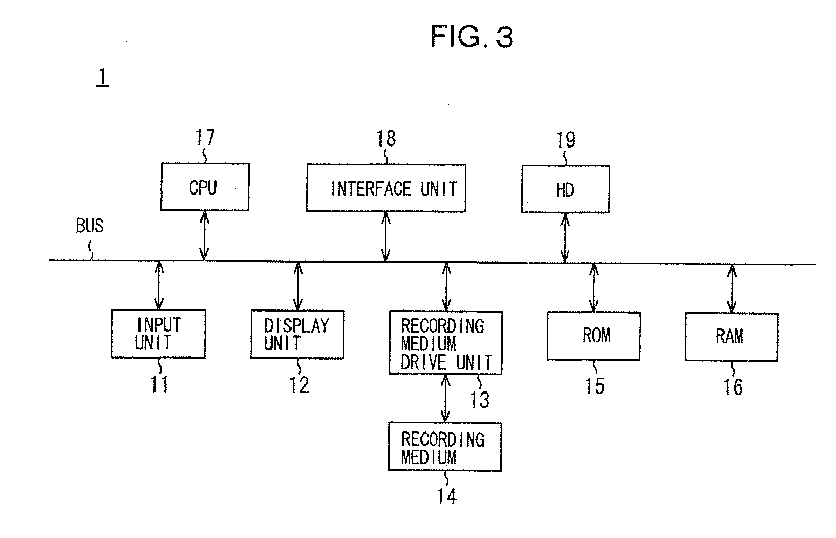

Hereinafter, the hardware configuration of an example of an information processing device 1 will be described with reference to Fig. 3. Fig. 3 is a hardware block diagram of an example of the information processing device. As shown in Fig. 3, the information processing device 1 has, as the hardware configuration, an input unit 11, a display unit 12, a recording medium drive unit 13, a ROM (Read Only Memory) 15, a RAM (Random Access Memory) 16, a CPU (Central Processing Unit) 17, an interface unit 18, and a HD (Hard Disk) 19.

The input unit 11 includes a keyboard, a mouse, and so on operated by an operator (or a user) of the information processing device 1 and is used for inputting various kinds of operation information and so on to the information processing device 1. The display unit 12 includes a display and so on used by the operator of the information processing device 1 and is used for displaying various kinds of information (or screens) and so on. The interface unit 18 is an interface connecting the information processing device 1 to a network and the like.

A pathway display program for displaying a pathway on a screen or the like is supplied to the information processing device 1 from a recording medium 14 such as, for example, a CD-ROM, or is downloaded via a network or the like. The recording medium 14 is set in the recording medium drive unit 13, and the pathway display program is installed into the HD 19 from the recording medium 14 via the recording medium drive unit 13.

In the ROM 15, a program and the like first read upon power-on of the information processing device 1 are recorded. The RAM 16 is a main memory of the information processing device 1. When necessary, the CPU 17 reads the pathway display program from the HD 19 to store it in the RAM 16 and executes the pathway display program, thereby providing all or part of later-described functions and executing later-described flowcharts and so on. The HD 19 stores, besides the pathway display program, for example, pathway information, association information, expression information, and so on which will be described later. Incidentally, all or some of the pathway information, the association information, the expression information, and so on may be stored in a HD of another device connected to the information processing device 1 via a network, or the like. However, to simplify the description, the following description will be given on assumption that the pathway information, the association information, the expression information, and so on are stored in the HD 19.

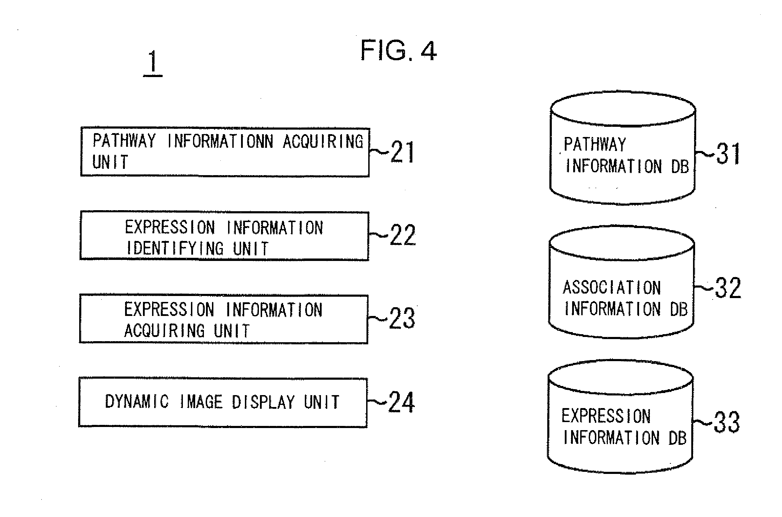

Next, the functional configuration of an example of the information processing device 1 is shown in Fig. 4. Fig. 4 is a functional block diagram of an example of the information processing device (No. 1). As shown in Fig. 4, the information processing device 1 has, as the functional configuration, a pathway information acquiring unit 21, an expression information identifying unit 22, an expression information acquiring unit 23, a dynamic image display unit 24, a pathway information DB 31, an association information DB 32, and an expression information DB 33.

The pathway information acquiring unit 21 acquires pathway information from the pathway information DB 31 in response to a user's request or the like via, for example, the input unit 11 or the like. The expression information identifying unit 22 identifies expression information corresponding to the pathway information acquired by the pathway information acquiring unit 21, based on the association information stored in the association information DB 32.

The expression information acquiring unit 23 acquires, from the expression information DB 33, the expression information identified by the expression information identifying unit 22. The dynamic image display unit 24 displays a pathway on a screen or the like displayed on the display unit 12, by using the pathway information acquired by the pathway information acquiring unit 21 and the expression information acquired by the expression information acquiring unit 23, and displays a dynamic image by changing the display of the pathway in time series. The dynamic image display unit 24 changes the size of each displayed node in time series according to, for example, an absolute value of a later-described expression value (a value of an expression amount), and changes color of each displayed node in time series according to, for example, a ratio of the expression value to an initial value. Since the dynamic image display unit 24 or the like changes, for example, the size of each displayed node according to the absolute value of the expression value in time series and changes the color of each displayed node according to the ratio of the expression value to the initial value, a user can easily get a visual idea of processes of the expression of proteins, mRNA, and the like that occurs in time series. Incidentally, the dynamic image display unit 24 may change the color of each displayed node in time series according to the absolute value of the expression value and may change the size of each displayed node in time series according to the ratio of the expression value to the initial value. However, to simplify the description, the description below will be given on assumption that the dynamic image display unit 24 changes the size of each displayed node in time series according to the absolute value of the expression value and changes the color of each displayed node in time series according to the ratio of the expression value to the initial value.

As will be described later, the dynamic image display unit 24 displays two pathways on one screen according to a user's request or the like given via, for example, the input unit 11 or the like and displays dynamic images by changing the display of the pathways in time series. In this manner, for example, the dynamic image display unit 24 or the like displays the two pathways on one screen, for example, one being a pathway in which nodes represent proteins of a normal cell (normal cell) and the other being a pathway in which nodes represent proteins of a cancer cell, and consequently, a user can easily get a visual idea of a difference between processes of the expression of the proteins, mRNA, and the like that occurs in the two pathways in time series. Incidentally, the dynamic image display unit 24 may display, on one screen, two pathways of proteins, mRNA, and the like for different tissues, organs, medications, reagents, differentias, developments (egg, fetus, adjust animal), or the like, according to a user's request or the like given via, for example, the input unit 11 or the like.

The dynamic image display unit 24 may extract a node selected by, for example, a user or the like among nodes displayed in the pathway (for example, erase the display of other nodes and so on or pop-up display the selected node), and change the size and color of the node in time series, in response to a user's request or the like given via, for example, the input unit 11 or the like. If the dynamic image display unit 24 thus extracts and displays a specific node, it is possible to show in detail a change of a node of interest such as a node which presents a rapid change.

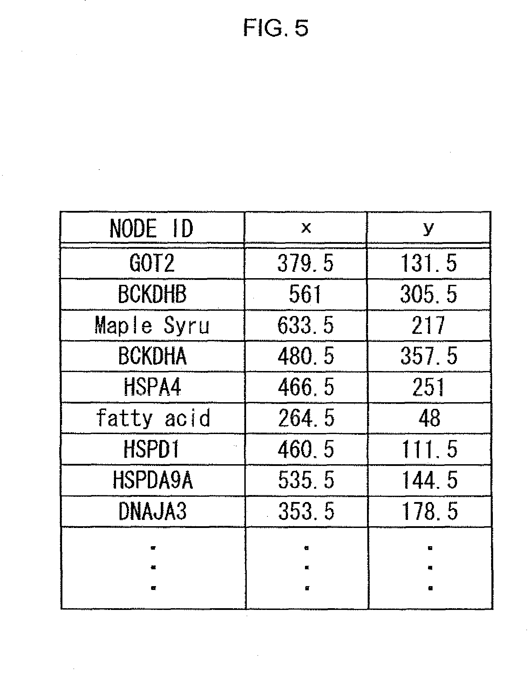

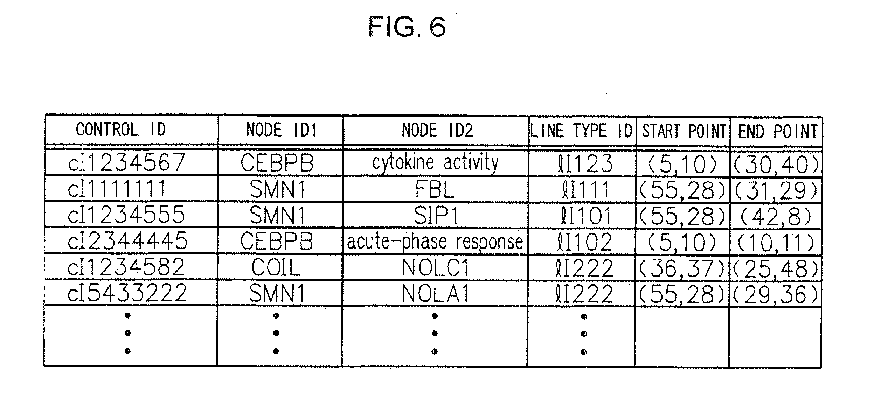

The pathway information DB 31 manages the pathway information. The pathway information includes pathway ID identifying each pathway, pathway attribute information indicating the attribute of each pathway, pathway data used for visually displaying each pathway, node attribute information indicating the attribute of nodes (for example, whether the shape of the nodes is circular, elliptical, polygonal, or the like), control attribute information indicating the attribute of controls (for example, whether the form of the controls is a solid line, a dotted line, or the like), and so on. The pathway data includes information regarding the controls and the nodes forming the pathway. The control information includes control position information. The node information includes node position information and data regarding an expression amount of a protein, mRNA, and the like corresponding to each of the nodes. The data regarding the expression amount corresponds to an initial value of later-described expression data. An example of the node position information is shown in Fig. 5, which will be described later. An example of the control attribute information is shown in Fig. 6, which will be described later.

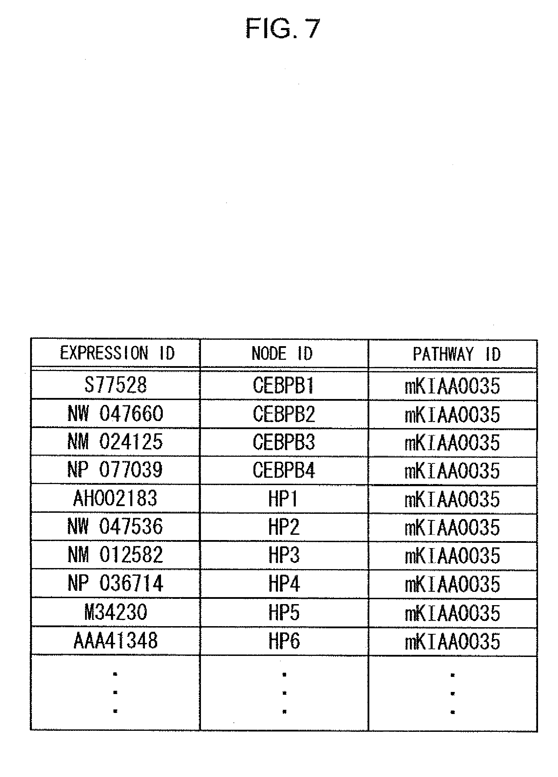

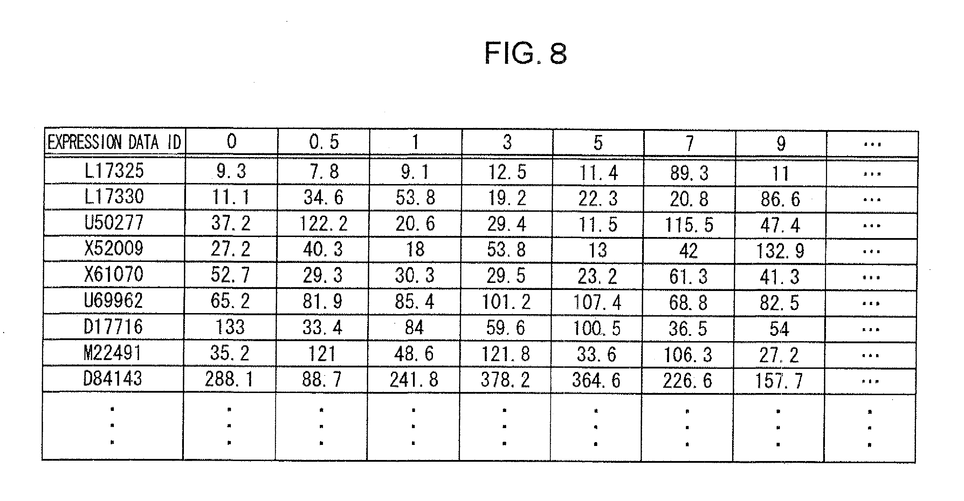

The association information DB 32 manages the association information associating the pathway information and the expression information with each other. An example of the association information is shown in Fig. 7, which will be described later. The expression information DB 33 manages the expression information which is pathway information at each instant after a predetermined period of time passes from the time when the pathway information has the initial values and which indicates how the pathway changes in accordance with the expression of proteins and the like. The expression information includes expression ID identifying the expression information, expression attribute information indicating the attribute of the expression information, the later-described expression data, and so on. An example of the expression data is shown in Fig. 8, which will be described later.

Next, an example of the node position information is shown in Fig. 5. Fig. 5 is a chart showing an example of the node position information. As shown in Fig. 5, the node position information includes node ID identifying each node and an x coordinate and a y coordinate of each node.

Next, an example of the control attribute information is shown in Fig. 6. Fig. 6 is a chart showing an example of the control attribute information. As shown in Fig. 6, the control attribute information includes control ID identifying each control, node ID1 and node ID2 identifying nodes, line type ID identifying the type of a line of each control, start point, and end point. Here, the coordinates of a start point (coordinate of the node ID1) are stored in the start point. The coordinates of an end point (coordinates of the node ID2) are stored in the end point.

Next, an example of the association information is shown in Fig. 7. Fig. 7 is a chart showing an example of the association information. As shown in Fig. 7, the association information includes expression ID, node ID, and pathway ID. In this embodiment, to simplify the description, the description will be given on assumption that the names of nodes identified by the node IDs and the names of, for example, proteins, mRNA, and the like are in one-to-one correspondence. The same applies to the following.

Next, an example of the expression data is shown in Fig. 8. Fig. 8 is a chart showing an example of the expression data. As shown in Fig. 8, the expression data includes expression data ID identifying expression data and values of an expression amount (expression values) at every instant after a predetermined time passes. Incidentally, a unit of the predetermined time shown in Fig. 8 may be second, minute, hour, day, or the like.

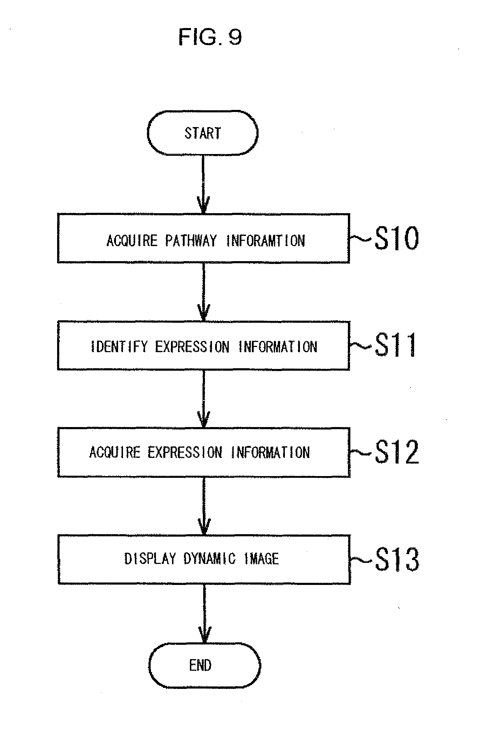

Next, an example of pathway display processing is shown in Fig. 9. Fig. 9 is a flowchart showing an example of the pathway display processing. At Step S10, the pathway information acquiring unit 21 acquires pathway information from the pathway information DB 31 in response to a user's request or the like given via, for example, the input unit 11 or the like.

After Step S10, the flow goes to Step S11, where, based on the association information stored in the association information DB 32, the expression information identifying unit 22 identifies expression information corresponding to the pathway information which is acquired at Step S10 by the pathway information acquiring unit 21.

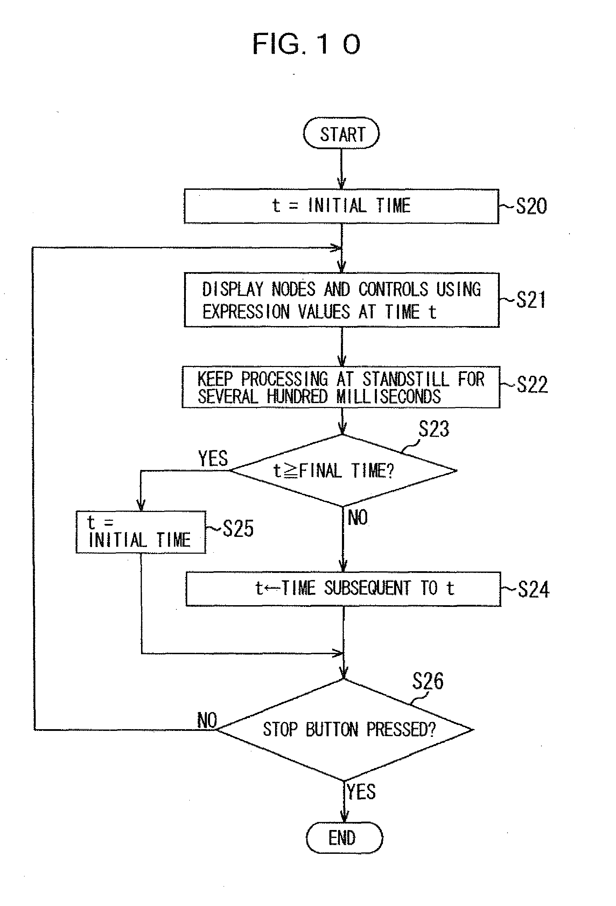

After Step S11, the flow goes to Step S12, where the expression information acquiring unit 23 acquires, from the expression information DB 33, the expression information which is identified at Step S11 by the expression information identifying unit 22. After Step S12, the flow goes to Step S13, where the dynamic image display unit 24 displays a pathway on a screen or the like displayed on the display unit 12, by using the pathway information acquired by the pathway information acquiring unit 21 and the expression information acquired by the expression information acquiring unit 23, and displays a dynamic image by changing the display of the pathway. A detail of dynamic image display processing by the dynamic image display unit 24 at Step S13 is shown in Fig. 10, which will be described later.

As a result of the information processing device 1 performing the processing as shown in Fig. 9, a user can easily get a visual idea of processes of the expression of proteins, mRNA, and the like that occurs in time series.

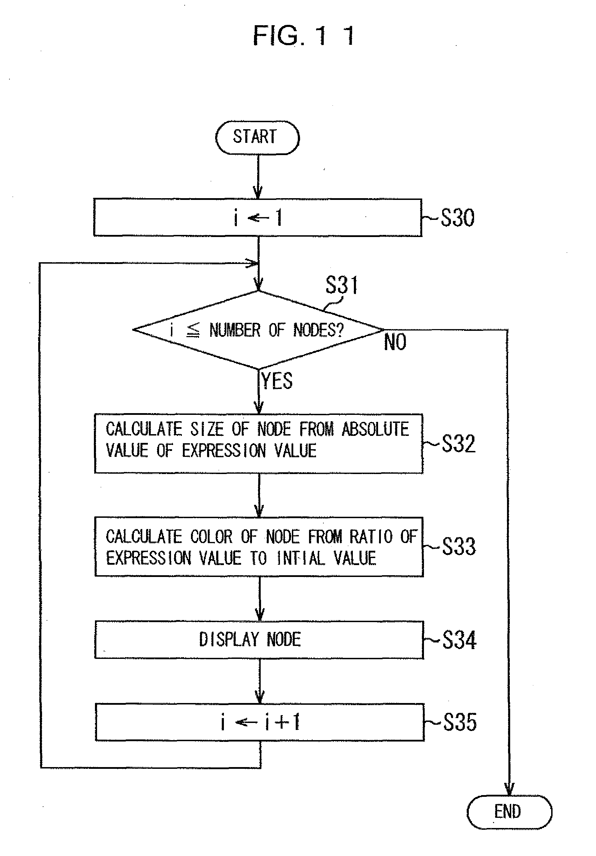

Next, an example of the dynamic image display processing is shown in Fig. 10. Fig. 10 is a flowchart showing an example of the dynamic image display processing (No. 1). At Step S20, the dynamic image display unit 24 substitutes initial time in a variable t. At Step S21, the dynamic image display unit 24 displays nodes, controls, and so on by using expression values at time t. Details of display processing of the nodes and so on using the expression values by the dynamic image display unit 24 at Step S21 is shown in Fig. 11, which will be described later.

After Step S21, the dynamic image display unit 24 goes to Step S22 to keep the processing at standstill for several hundred milliseconds. After Step S22, the dynamic image display unit 24 goes to Step S23 to determine whether or not the time substituted in the variable t is equal to or more than final time which is, for example, determined in advance. The dynamic image display unit 24 goes to Step S25 when determining that the time substituted in the variable t is equal to or more than the final time which is, for example, determined in advance (YES at Step S23), while going to Step S24 when determining that the time substituted in the variable t is less than the final time which is, for example, determined in advance.

At Step S24, the dynamic image display unit 24 substitutes time subsequent to the time currently substituted in the variable t.

At Step S25, the dynamic image display unit 24 substitutes the initial time in the variable t and goes to Step S26. At Step S26, the dynamic image display unit 24 determines whether or not a Stop button displayed on the screen has been pressed by a user or the like. The dynamic image display unit 24 finishes the dynamic image display processing when determining that the Stop button has been pressed by the user or the like (YES at Step S26), while returning to the process at Step S21 when determining that the Stop button has not been pressed (NO at Step S26).

As a result of the dynamic image display unit 24 performing the processing shown in Fig. 10, the expression processes of proteins, mRNA, and the like can be displayed by a dynamic image.

Next, an example of the display processing of nodes and so on is shown in Fig. 11. Fig. 11 is a flowchart showing an example of the display processing of the nodes and so on (No. 1).

At Step S30, the dynamic image display unit 24 substitutes 1 in a variable i. At Step S31, the dynamic image display unit 24 determines whether or not the value of the variable i is equal to or less than the number of nodes included in the pathway as a display target. The dynamic image display unit 24 refers to the pathway information managed in the pathway information DB 31 or the like. The dynamic image display unit 24 goes to Step S32 when determining that the value of the variable i is equal to or less than the number of the nodes included in the pathway as a display target (YES at Step S31), while finishing the display processing of the nodes and so on using the expression values when determining that the value of the variable i is more than the number of the nodes included in the pathway as a display target (NO at Step S31).

At Step S32, the dynamic image display unit 24 calculates the size of a node to be displayed from an absolute value of the expression value. For example, the dynamic image display unit 24 makes the size of a node included in the pathway as a display target large if the absolute value of its expression value is larger than absolute values of expression values of the other nodes included in the same pathway, and makes the size of a node small if the absolute value of its expression value is smaller than the absolute values of the expression values of the other nodes included in the same pathway. After Step S32, the dynamic image display unit 24 goes to Step S33 to calculate the color of the node to be displayed from a ratio of the expression value to an initial value. For example, as a result of the ratio calculation, the dynamic image display unit 24 colors the node red if the ratio of the expression value of the node to the initial value is large (for example, if the ratio is two times or more), colors the node black if the ratio indicates no change (for example, if the ratio is about one time), and colors the node green if the ratio is small (for example, if the ratio is 0.5 times or less).

After Step S33, the dynamic image display unit 24 goes to Step S34 to display the node according to the node size calculated at Step S32 and the node color calculated at Step S33. After Step S34, the dynamic image display unit 24 goes to Step S35 to increment the variable i and goes to Step S31. For example, when 1 is substituted in the variable i, the dynamic image display unit 24 may display a control, though not shown in Fig. 11 to simplify the description, as well as the node at Step S34 and so on. Further, for example, when 2 or more is substituted in the variable i, the dynamic image display unit 24 may erase the display of the control related to the node at Step S34 and so on, according to the node size (that is, the absolute value of the expression value) or the like calculated at Step S32.

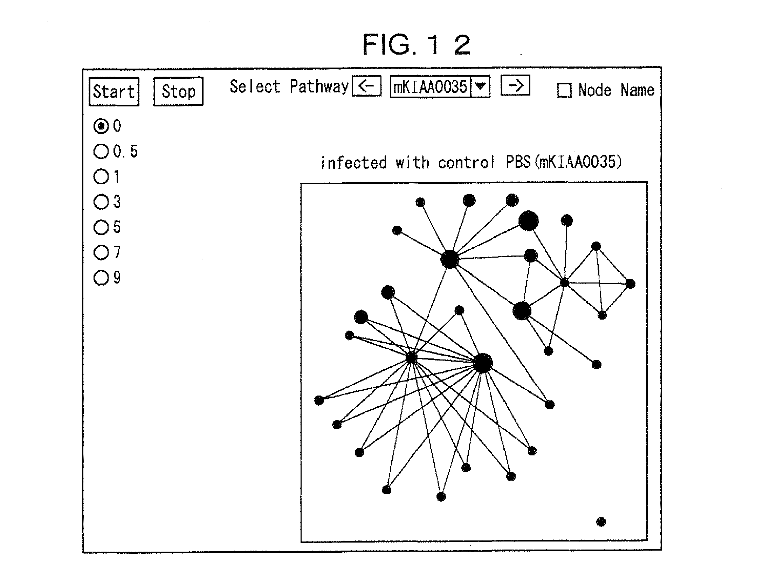

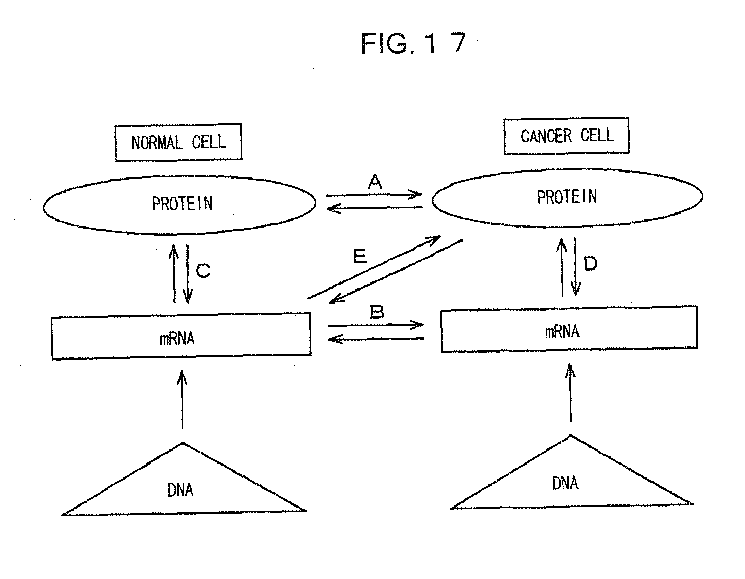

Here, even if a node represents, for example, a protein, the expression value (or the expression information) based on which the dynamic image display unit 24 calculates the node size and color is not limited to the expression value (or the expression information) of a protein corresponding to the node. In calculating the node size and color, the dynamic image display unit 24 sometimes uses the expression value (or the expression information) or the like of mRNA of the protein, according to a user's request given via, for example, the input unit 11 or the like. An advantage and so on of thus using the expression value of mRNA and the like of the protein in calculating the size and color of the node corresponding to the protein will be described later with reference to Fig. 17 and so on. Further, examples of nodes and controls (that is, a pathway) displayed by the dynamic image display unit 24 are shown in FIG. 12 to Fig. 16 and so on, which will be described later.

As a result of the dynamic image display unit 24 performing the processing as shown in Fig. 11, it is possible to, for example, show a change of the expression of proteins or mRNA by the node size and color, which enables a user to easily get a visual idea of the processes of the expression of the protein, mRNA, and the like that occurs in time series. In particular, as a result of the dynamic image display unit 24 calculating the size of each node to be displayed from the absolute value of the expression value and displaying the node, it is possible for a user to, for example, easily see at how much ratio the expression value of some protein, mRNA and the like exists on the pathway as a display target, compared with expression values of other proteins, mRNA and the like. Further, as a result of the dynamic image display unit 24 calculating the color of each node to be displayed from a ratio of the expression value to the initial value and displaying the node, it is possible for a user to, for example, easily see by how much ratio some protein, mRNA, and the like has increased or decreased.

Further, as a result of the dynamic image display unit 24 calculating the size of each node to be displayed from the absolute value of the expression value and calculating the color of each node to be displayed from a ratio of the expression value to the initial value, thereby displaying the node while simultaneously changing its size and color, it is possible for a user to, for example, easily get a visual idea of how the expression values of proteins (or mRNA) and the like included in the pathway as a display target change with time.

Next, an example of a pathway display screen is shown in Fig. 12. Fig. 12 is a view showing the pathway display screen (No. 1). On the screen shown in Fig. 12, a pathway at time 0 is displayed. Nodes are displayed in black at the time 0.

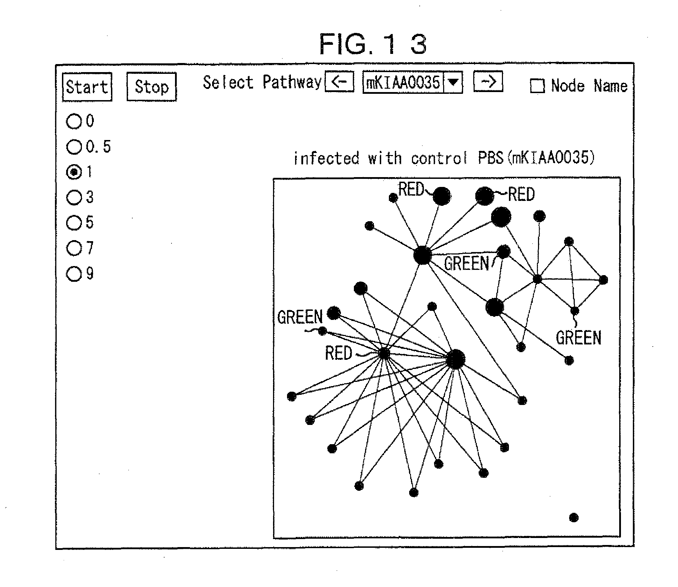

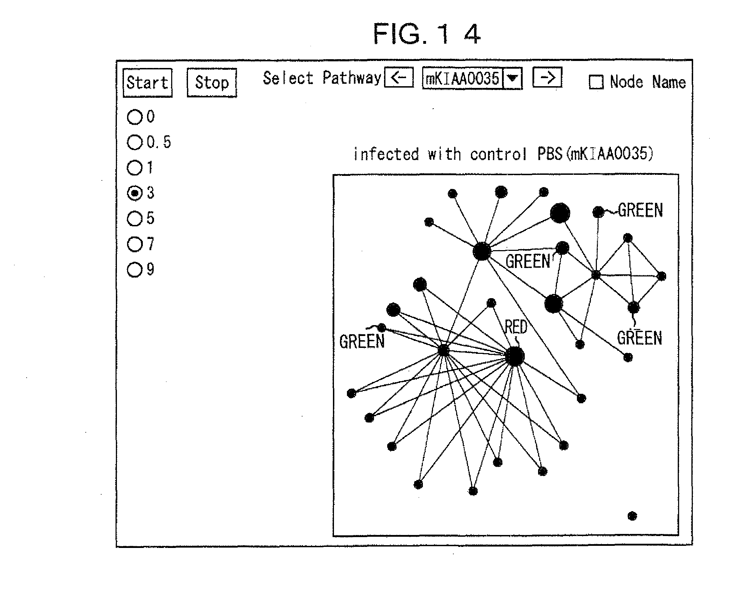

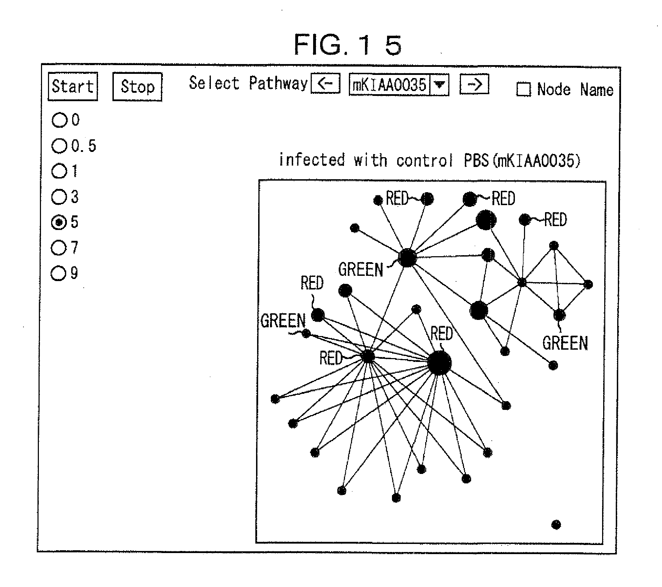

Next, other examples of the pathway display screen are shown in Fig. 13 to Fig. 15. Fig. 13 is a view showing the pathway display screen (No. 2). On the screen shown in Fig. 13, the pathway at time 1 is displayed. On the screen shown in Fig. 13, color and size of the nodes are changed from those on the screen shown in Fig. 12. Fig. 14 is a view showing the pathway display screen (No. 3). On the screen shown in Fig. 14, the pathway at time 3 is displayed. Fig. 15 is a view showing the pathway display screen (No. 4). On the screen shown in Fig. 15, the pathway at time 5 is displayed. As shown in Fig. 12 to Fig. 15 and so on, since the dynamic image display unit 24 displays processes of the expression of proteins, mRNA, and the like that occurs in time series, by a dynamic image, a user can easily get a visual idea of the expression processes.

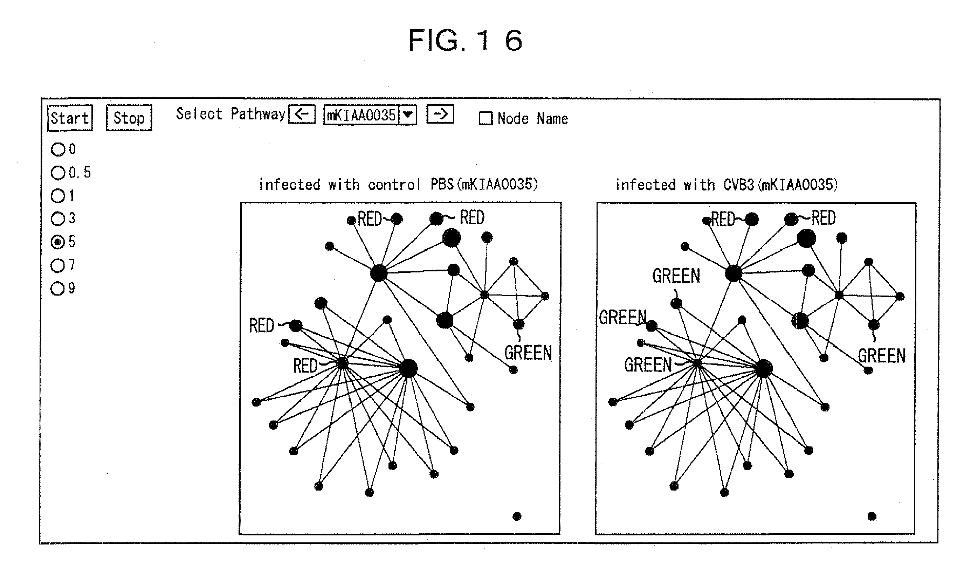

Next, another example of the pathway display screen is shown in Fig. 16. Fig. 16 is a view showing the pathway display screen (No. 5). Fig. 12 to Fig. 15 described above show the examples where the dynamic image display unit 24 displays one pathway on one screen, but the dynamic image display unit 24 may display two pathways on one screen as shown in Fig. 16, in response to a user's request or the like given via, for example, the input unit 11 or the like. As described above, if the dynamic image display unit 24 or the like displays two pathways on one screen, for example, one being a pathway in which nodes represent proteins of a normal cell and the other being a pathway in which nodes represent proteins of a cancer cell, a user can easily get a visual idea of the processes of the expression of the proteins, mRNA, and the like that occurs in time series, while comparing the pathway in which the nodes represent the proteins of the normal cell and the pathway in which the nodes represent the proteins of the cancer cell.

Next, examples of a display pattern (or a comparison pattern) in a case where the dynamic image display unit 24 displays two pathways on one screen are shown in Fig. 17. Here, as prerequisite knowledge, it has been known that an expression amount of mRNA and an expression amount of a protein are not necessarily correlated to each other. However, it is sure that the expression of mRNA occurs before the expression of a protein as a dependency relation. Further, it has been known that some protein A accelerates/inhibits the expression of a protein B. Further, mRNA or a protein once expressed is not necessarily accumulated in an organism but is decomposed. Therefore, there occurs some case where the expression cannot be confirmed on the mRNA level even though the expression is confirmed on the protein level.

Fig. 17 is a chart showing examples of the display pattern. One example of the display pattern is a pattern displaying a pathway in which nodes represent proteins of a normal cell and the color and size of the nodes are displayed by using expression values of the proteins, and a pathway in which nodes represent proteins of a cancer cell and the color and size of the nodes are displayed by using expression values of the proteins, as shown in A in Fig. 17. The display as shown in A in Fig. 17 performed by the dynamic image display unit 24 enables a user, for example, to compare the expression amounts of the proteins in the normal cell and the cancer cell in time series.

Another example of the display pattern is a pattern displaying a pathway in which nodes represent mRNA of a normal cell and the color and size the nodes are displayed by using expression values of the mRNA, and a pathway in which nodes represent mRNA of a cancer cell and the color and size of the nodes are displayed by using expression values of the mRNA, as shown in B in Fig. 17. The display as shown in B in Fig. 17 performed by the dynamic image display unit 24 enables a user, for example, to compare the expression amounts of the mRNA in the normal cell and the cancer cell in time series. The display shown in B in Fig. 17 is suitable especially for use in analyzing a defect at Step (A) shown in Fig. 2.

Still another example of the display pattern is a pattern displaying a pathway in which nodes represent proteins of a normal cell and the color and size of the nodes are displayed by using expression values of the proteins, and a pathway in which nodes represent the proteins of the normal cell and the color and size of the nodes are displayed by using expression values of mRNA of the proteins, as shown in C in Fig. 17. The display as shown in C in Fig. 17 performed by the dynamic image display unit 24 enables a user, for example, to compare expression patterns of the proteins and the mRNA in the normal cell when the expression step shown in Fig. 2 normally works.

Yet another example of the display pattern is a pattern displaying a pathway in which nodes represent proteins of a cancer cell and the color and size of the nodes are displayed by using expression values of the proteins, and a pathway in which nodes represent the proteins of the cancer cell and the color and size of the nodes are displayed by using expression values of mRNA of the proteins, as shown in D in Fig. 17. The display as shown in D in Fig. 17 performed by the dynamic image display unit 24 enables a user, for example, to analyze in which of the steps (A) to (C) shown in Fig. 2 abnormality occurred, in a case where a pathognomonic molecule or the like can be specified in the expression amount in the cancer cell when the cancer cell is compared with the normal cell by using the display shown in A in Fig. 17 and/or B in Fig. 17.

Yet another example of the display pattern is a pattern displaying a pathway in which nodes represent proteins of a cancer cell and the size and the color of the nodes are displayed by using expression values of the proteins, and a pathway in which nodes represent proteins of a normal cell and the color and size of the nodes are displayed by using expression values of mRNA of the proteins, as shown in E in Fig. 17. The display as shown in E in Fig. 17 performed by the dynamic image display unit 24 enables a user, for example, to analyze in which of the steps shown in Fig. 2 abnormality occurred, based on a change of the expression amounts of the proteins in the cancer cell, by using, as a reference, the other pathway where the expression amounts of the mRNA, its copy (replication) sequence and timing, and so on are normal. This is because in the pathway of the cancer cell, some protein is lacking or its expression amount is excessively large in some case. If a protein is lacking, a user can infer that abnormality occurred in an organism at and after the expression timing of the corresponding mRNA. Conversely, if the expression amount of a protein is excessively large, a user can infer that abnormality occurred at and after the expression timing of mRNA corresponding to an expression inhibiting protein. The display shown in E in FIG. 17 is a display suitable for use in analyzing a defect in steps (B) and (C) shown in Fig. 2.

As described above, according to this embodiment, processes of the expression of proteins, mRNA, and the like that occurs in time series can be displayed by dynamic images in the same or different time series. Therefore, a user can easily get a visual idea of the expression processes of amino acids and the like.

### (Embodiment 2)

The above embodiment 1 describes the function, method, and so on which makes it possible to easily get a visual idea of the processes of the expression of proteins, mRNA, and the like that occurs in time series, but by using the similar function, method, and so on, it is made possible to easily get a visual idea of processes of the generation of metabolites that occurs in time series.

The embodiment 2 described below shows the function, method, and so on of the present invention which makes it possible to easily get a visual idea of the generation processes of metabolites. The description of the embodiment 2 is almost the same as the above description except in that "proteins, mRNA, and the like" shown in the embodiment 1 is replaced by "metabolites", and "expression" shown in the embodiment 1 is replaced by "generation", and therefore detailed description will be omitted.

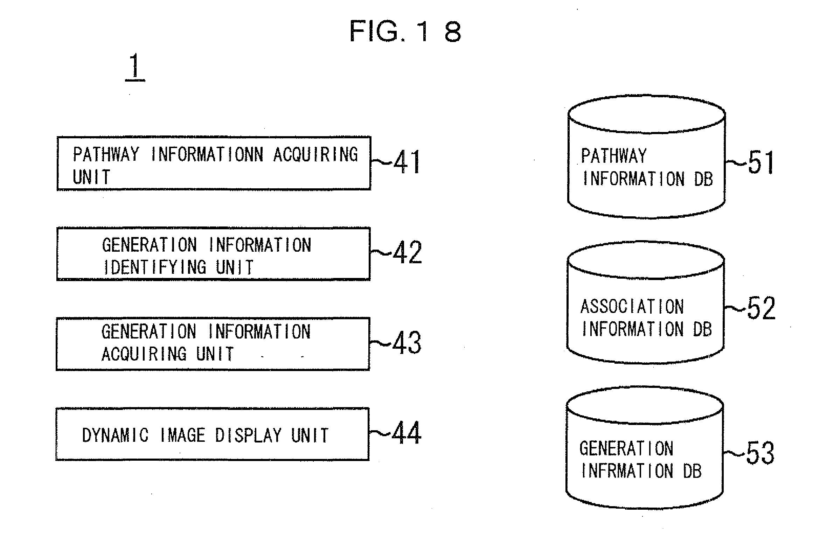

Next, the functional configuration of an example in this embodiment of the information processing device 1 is shown in Fig. 18. Fig. 18 is a functional block diagram of an example of the information processing device (No. 2). As shown in Fig. 18, the information processing device 1 has, as the functional configuration, a pathway information acquiring unit 41, a generation information identifying unit 42, a generation information acquiring unit 43, a dynamic image display unit 44, a pathway information DB 51, an association information DB 52, and a generation information DB 53.

The pathway information acquiring unit 41 acquires pathway information from the pathway information DB 51, in response to a user's request or the like via, for example, the input unit 11 or the like. Based on association information stored in the association information DB 5, the generation information identifying unit 42 identifies generation information corresponding to the pathway information acquired by the pathway information acquiring unit 41.

The generation information acquiring unit 43 acquires, from the generation information DB 53, the generation information identified by the generation information identifying unit 42. The dynamic image display unit 44 displays a pathway on a screen or the like displayed on the display unit 12, by using the pathway information acquired by the pathway information acquiring unit 41 and the expression information acquired by the generation information acquiring unit 43, and displays a dynamic image by changing the display of the pathway in time series. The dynamic image display unit 44 changes the size of each displayed node in time series according to, for example, an absolute value of a later-described generation value (a value of a generation amount of a metabolite), or changes color of each displayed node in time series according to, for example, a ratio of the generation value to an initial value. Since, for example, the dynamic image display unit 24 or the like changes the size of each displayed node in time series according to the absolute value of the generation value and changes the color of each displayed node in time series according to the ratio of the generation value to the initial value, a user can easily get a visual idea of processes of the generation of metabolites and the like that occurs in time series. Incidentally, the dynamic image display unit 44 may change the color of each displayed node in time series according to the absolute value of the generation value and may change the size of each displayed node in time series according to the ratio of the generation value to the initial value. However, to simplify the description, the description below will be given on assumption that the dynamic image display unit 44 changes the size of each displayed node in time series according to the absolute value of the generation value and changes the color of each displayed node in time series according to the ratio of the generation value to the initial value.

The pathway information DB 51 manages the pathway information. The pathway information includes pathway ID identifying each pathway, pathway attribute information indicating the attribute of each pathway, pathway data used for visually displaying each pathway, node attribute information indicating the attribute of nodes (for example, whether the shape of the nodes is circular, elliptical, polygonal, or the like), control attribute information indicating the attribute of controls (for example, whether the form of the controls is a solid line, a dotted line, or the like), and so on. The pathway data includes information regarding the controls and the nodes forming the pathway. The control information includes control position information. The node information includes node position information and data regarding a generation amount of a metabolite and the like corresponding to each of the nodes. The data regarding the generation amount corresponds to an initial value of later-described generation data.

The association information DB 52 manages the association information associating the pathway information and the generation information with each other. The generation information DB 53 manages the generation information which is pathway information at each instant after a predetermined period of time passes from the time when the pathway information has the initial values, and which indicates how the pathway changes in accordance with the generation of the metabolites and the like. The generation information includes generation ID identifying the generation information, generation attribute information indicating the attribute of the generation information, generation data which is data regarding the generation amounts of the metabolites and the like at each instant after a predetermined time passes, and so on.

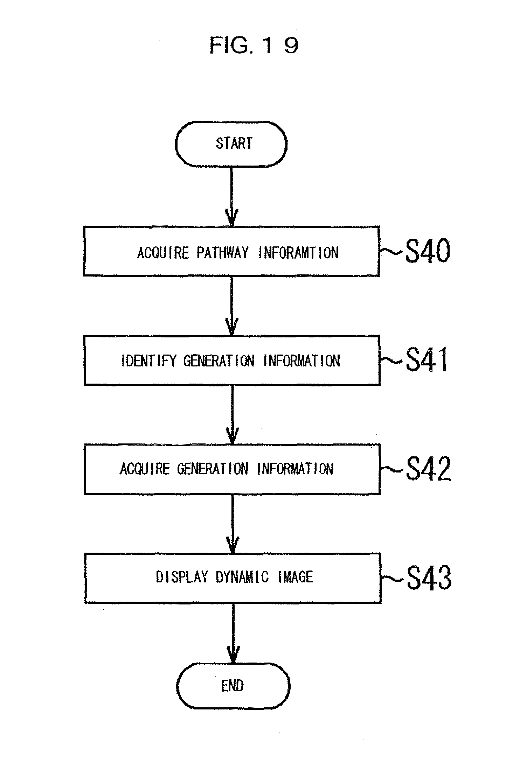

Next, an example in this embodiment of the pathway display processing is shown in Fig. 19. Fig. 19 is a flowchart showing an example of the pathway display processing (No. 2). At Step S40, the pathway information acquiring unit 41 acquires pathway information from the pathway information DB 51 in response to a user's request or the like given via, for example, the input unit 11 or the like.

After the Step S40, the flow goes to Step S41, where, based on the association information stored in the association information DB 5, the generation information identifying unit 42 identifies generation information corresponding to the pathway information which is acquired at Step S40 by the pathway information acquiring unit 41.

After Step S41, the flow goes to Step S42, where the generation information acquiring unit 43 acquires, from the generation information DB 53, the generation information which is identified at Step S41 by the generation information identifying unit 42. After Step S42, the flow goes to Step S43, where the dynamic image display unit 44 displays a pathway on a screen or the like displayed on the display unit 12, by using the pathway information acquired by the pathway information acquiring unit 41 and the generation information acquired by the generation information acquiring unit 43, and displays a dynamic image by changing the display of the pathway.

As a result of the information processing device 1 performing the processing shown in Fig. 19, a user can easily get a visual idea of processes of the generation of metabolites and the like that occurs in time series.

As described above, according to this embodiment, processes of the generation of metabolites and the like that occurs in time series can be displayed by dynamic images in the same or different time series. Therefore, a user can easily get a visual idea of the generation processes of the metabolites and the like.

### (Embodiment 3)

For example, the above-described embodiment 1 shows the example where the information processing device 1 displays, on one screen, pathways which are the same but uses different expression information, for example, one of them being a pathway in which nodes represent proteins of a normal cell and the other being a pathway in which nodes represent proteins of a cancer cell, and changes these pathways based on the same time series (for example, see Fig. 16 and so on).

However, the information processing device 1 may display a plurality of the same pathways on one screen, and change the displays of the respective pathways based on different time series.

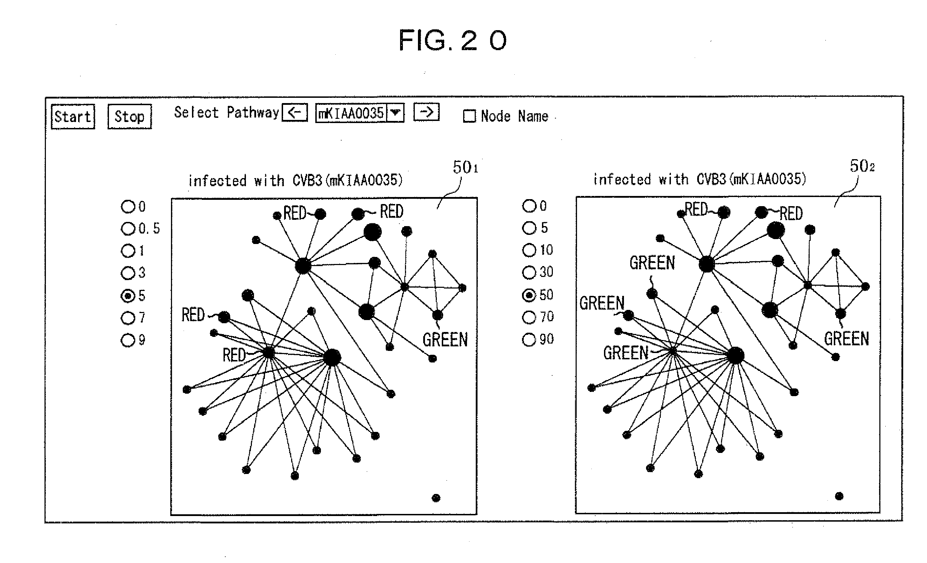

Fig. 20 is a view showing a pathway display screen (No. 6). In Fig. 20, 50 denotes a form. In this embodiment, the description will be given on assumption that the information processing device 1 displays a plurality of forms (50₁ and 50₂ in the example in Fig. 20) on the display screen in response to a user's request or the like, and displays one pathway in each of the forms.

The pathway shown in the form 50₁ and the pathway shown in the form 50₂ in Fig. 20 are, for example, pathways in which nodes represent proteins of the same cancer cell. However, the time series of the pathway shown in the form 50₁ is "0, 0.5, 1, 3, 5, 7, 9", while the time series of the pathway shown in the form 50₂ is "0, 5, 10, 30, 50, 70, 90". A unit of the time may be second, minute, hour, day, or the like as previously described.

The information processing device 1 stores, in, for example, the HD 19 or the like, the node position information shown in Fig. 5 of the embodiment 1, the control attribute information shown in Fig. 6, the association information shown in Fig. 7, the expression data shown in Fig. 8, and so on, in correspondence to each of the forms 50. The information processing device 1 executes the processing shown in Fig. 9 to Fig. 11 of the embodiment 1 for each of the forms 50 to display dynamic images.

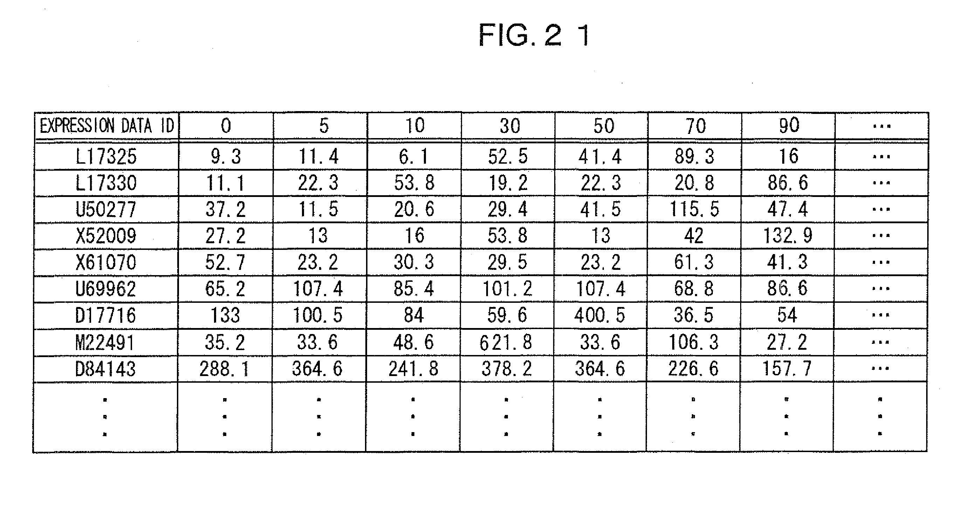

Here, an example of expression data corresponding to the pathway shown in the form 50₂ is shown in Fig. 21. Fig. 21 is a chart showing an example of the expression data (No. 2). As compared with the expression data shown in Fig. 8, a unit of the time passage is different in the expression data shown in Fig. 21.

The display as shown in Fig. 20 performed by the information processing device 1 enables a user, for example, to specify a protein which, in the short term, has a small expression amount and thus has little influence on the whole but which, in the long term, has a large expression amount and thus has great influence on the whole, a protein which conversely, in the short term, has a large expression amount and thus has great influence on the whole but which, in the long term, has a sharply decreased expression amount and thus has little influence on the whole, and so on.

As described above, according to this embodiment, it is made possible to easily get a visual idea of the expression processes of proteins, mRNA, and the like in different time series.

### (Embodiment 4)

For example, in the above-described embodiment 1, the information processing device 1 acquires the pathway information, identifies the expression information based on the acquired pathway information and the association information, acquires the identified expression information, and displays a dynamic image (see, for example, Fig. 9 and so on). That is, in the description of the embodiment 1, it is preconditioned that the pathway information and the expression information are always associated with each other in the association information, and the expression information corresponding to the pathway information is always stored in the expression information DB 33.

However, there may be a case where the pathway information and the expression information are not necessarily associated with each other in the association information, and a case where the expression information corresponding to the pathway information is not stored in the expression information DB 33. Such cases may possibly occur especially in the structure shown in a later-described embodiment, specifically, the structure in which a communication terminal device connected to the information processing device 1 via a network is permitted to edit a pathway, and the information processing device 1 receives the edit result from the communication terminal device to reflect the edit result in the pathway information DB 31 and/or the association information DB 32 and/or the expression information DB 33.

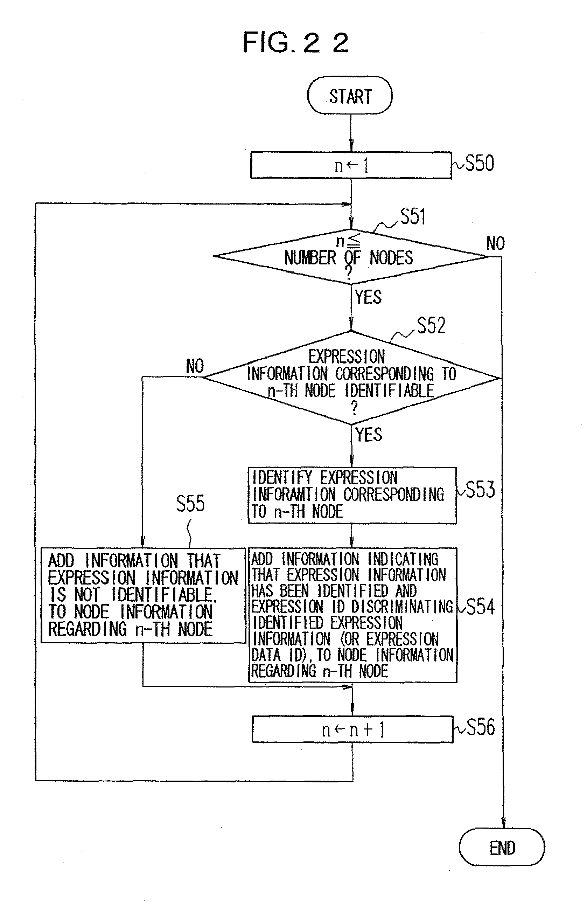

Next, an example in this embodiment of the expression information identification processing described previously at Step S11 and so on in Fig. 9 is shown in Fig. 22. Fig. 22 is a flowchart showing an example of the expression information identification processing. At Step S50, the expression information identifying unit 22 substitutes 1 in a variable n.

At Step S51, the expression information identifying unit 22 determines whether or not the value of the variable n is equal to or less than the number of nodes included in a pathway in a form as a display target. The expression information identifying unit 22 refers to the pathway information managed in the pathway information DB 31 or the like. The expression information identifying unit 22 goes to Step S52 when determining that the value of the variable n is equal to or less than the number of the nodes included in the pathway in the form as a display target (YES at Step S51), while finishing the expression information identification processing when determining that the value of the variable n is more than the number of the nodes included in the pathway in the form as a display target (NO at Step S51).

At Step S52, the expression information identifying unit 22 searches for corresponding association information as shown in Fig. 7 based on the node ID of a node corresponding to the value of the variable n and the pathway ID of the pathway in the form as a display target, and determines whether or not the expression information is identifiable, according to whether or not the expression ID corresponding to the node ID and the pathway ID exists. The expression information identifying unit 22 goes to Step S53 when determining that the expression information is identifiable (YES at Step S52), while going to Step S55 when determining that the expression information is not identifiable (NO at Step S52).

At Step S53, the expression information identifying unit 22 acquires the expression ID corresponding to the node ID and the pathway ID from the corresponding association information as shown in Fig. 7, based on the node ID of the node corresponding to the value of the variable n and the pathway ID of the pathway in the form as a display target, thereby identifying the expression information.

After Step S53, the expression information identifying unit 22 goes to Step S54 to add information indicating that the expression information has been identified and the expression ID discriminating the identified expression information (or the expression data ID discriminating the expression data included in the identified expression information), to the node information or the like regarding the node corresponding to the value of the variable n. To simplify the description, the following description will be given on assumption that the expression information identifying unit 22 adds not the expression ID but the expression data ID to the node information or the like. It should be noted that this does not restrict the embodiment of the present invention.

Meanwhile, at Step S55, the expression information identifying unit 22 adds information indicating that the expression information is not identifiable, to the node information or the like regarding the node corresponding to the value of the variable n.

At Step S56, the expression information identifying unit 22 increments the value of the variable n by one and goes to Step S51.

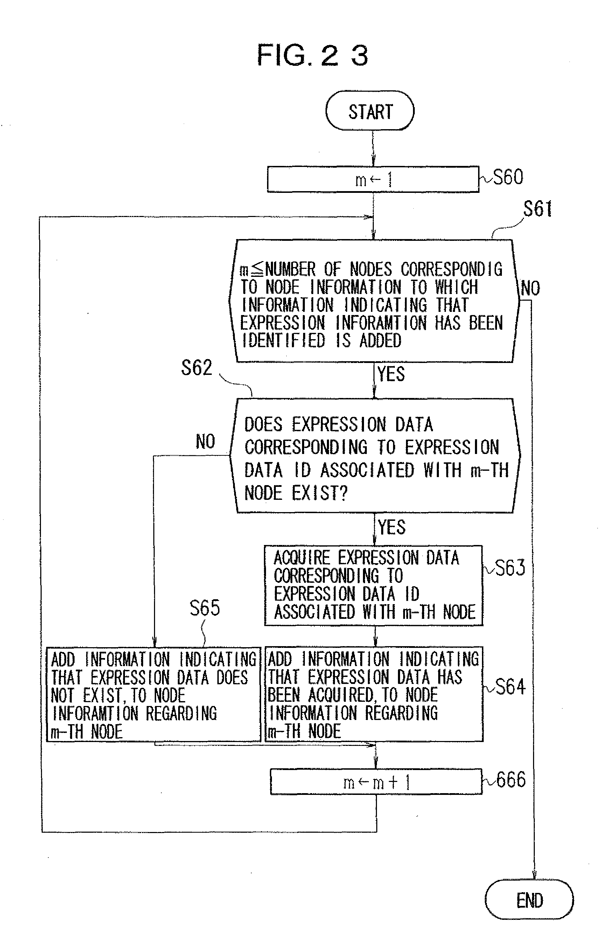

Next, an example in this embodiment of the expression information acquisition processing described above in Step S12 and so on in Fig. 9 is shown in Fig. 23. Fig. 23 is a flowchart showing an example of the expression information acquisition processing. The expression information acquiring unit 23 executes the following processing to nodes to whose node information or the like the information indicating that the expression information has been identified is added.

At Step S60, the expression information acquiring unit 23 substitutes 1 in a variable m. At Step S61, the expression information acquiring unit 23 determines whether or not the value of the variable m is equal to or less than the number of the nodes to whose node information or the like the information indicating that the expression information has been identified is added. The expression information acquiring unit 23 goes to Step S62 when determining that the value of the variable m is equal to or less than the number of the nodes to whose node information or the like the information indicating that the expression information has been identified is added (YES at Step S61), while finishing the expression information acquisition processing when determining that the value of the variable m is more than the number of the nodes to whose node information or the like the information indicating that the expression information has been identified is added (NO at Step S61).

At Step S62, the expression information acquiring unit 23 refers to the corresponding expression data as shown in Fig. 8, based on the expression data ID included in the node information or the like regarding a node corresponding to the value of the variable m, and determines whether or not the expression data exists, according to whether or not the expression data corresponding to the expression data ID exists. The expression information acquiring unit 23 goes to Step S63 when determining that the expression data exists (YES at Step S62), while going to Step S65 when determining that the expression data does not exist (NO at Step S62).

At Step S63, the expression information acquiring unit 23 acquires the expression data from the corresponding expression data as shown in Fig. 8, based on the expression data ID included in the node information or the like regarding the node corresponding to the value of the variable m. After Step S63, the expression information acquiring unit 23 goes to Step S64 to add information indicating that the expression data has been acquired, to the node information or the like regarding the node corresponding to the value of the variable m.

Meanwhile, at Step S65, the expression information acquiring unit 23 adds information indicating that the expression data does not exist, to the node information or the like regarding the node corresponding to the value of the variable m.

At Step S66, the expression information acquiring unit 23 increments the value of the variable m by one and goes to Step S61.

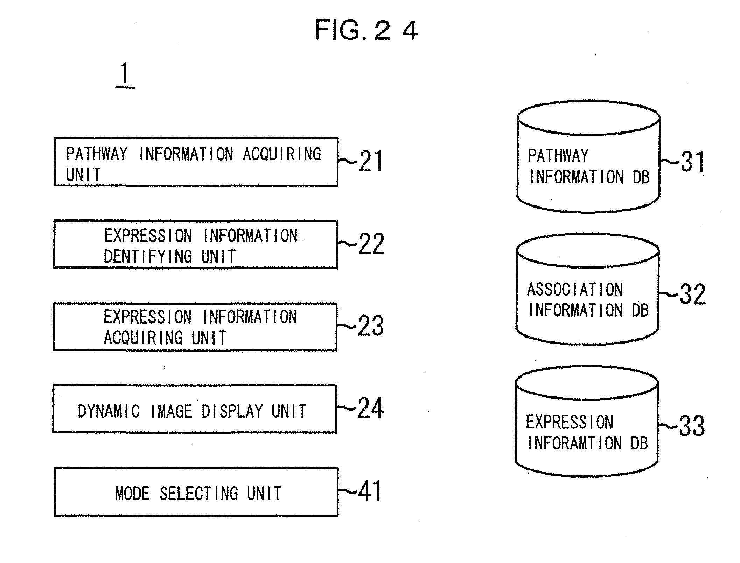

Next, the functional configuration of an example in this embodiment of the information processing device 1 is shown in Fig. 24. Fig. 24 is a functional block diagram of an example of the information processing device (No. 3). As shown in Fig. 24, the information processing device 1 has, as the functional configuration, a pathway information acquiring unit 21, an expression information identifying unit 22, an expression information acquiring unit 23, a dynamic image display unit 24, a mode selecting unit 41, a pathway information DB 31, an association information DB 32, and an expression information DB 33.

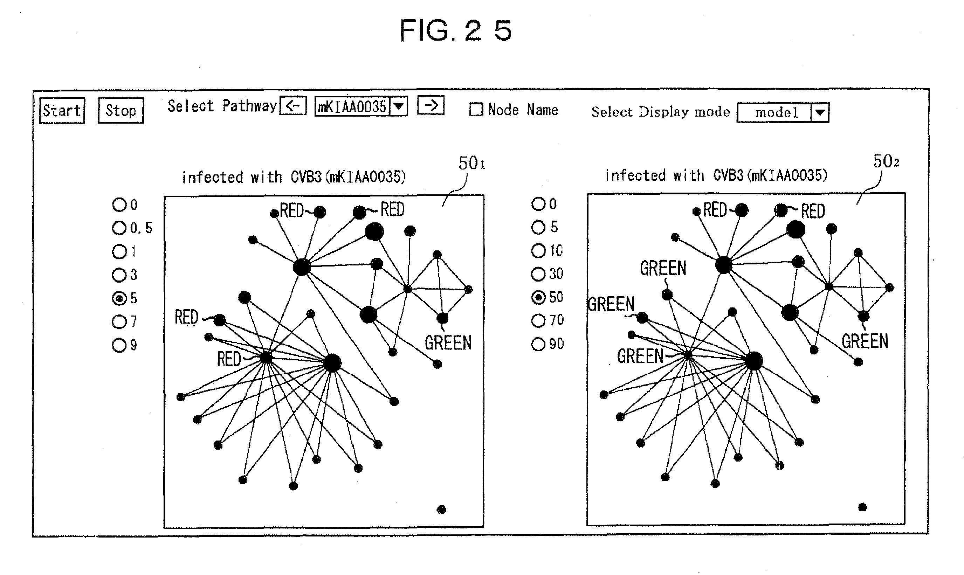

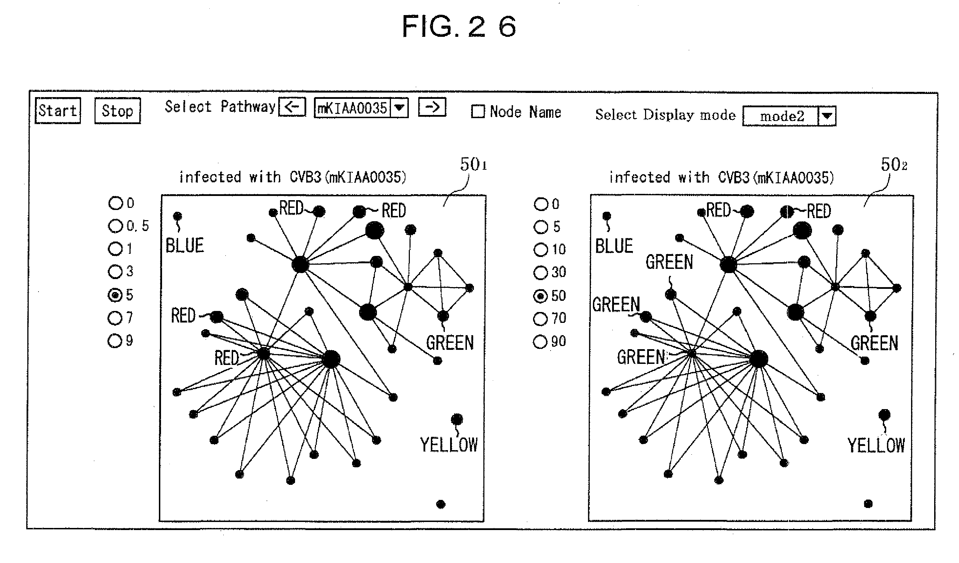

As compared with the functional configuration of the information processing device 1 shown in Fig. 4, the functional configuration of the information processing device 1 shown in Fig. 24 is different in that the mode selecting unit 41 is further included as the functional configuration of the information processing device 1. The mode selecting unit 41 selects a pathway display mode based on mode information stored in the RAM 16 or the like and notifies the selection result to the dynamic image display unit 24 and so on. Incidentally, in the mode information stored in the RAM 16 or the like, a default mode (for example, mode 1) is set in advance, and when a mode is selected by a user or the like on a display screen shown in Fig. 25 and Fig. 26 to be described later, the selected mode is set in the mode information.

To simplify the description, as examples of the pathway display mode, mode 1 and mode 2 are taken as an example. Specifically, a node regarding which the expression information is not identifiable in the expression information identification processing shown in Fig. 22 and a node regarding which the expression data is not acquirable in the expression information acquisition processing shown in Fig. 23 are not displayed in the mode 1 and are displayed in the mode 2.

Fig. 25 is a view showing a pathway display screen (No. 6). Fig. 26 is a view showing a pathway display screen (No. 7). Fig. 25 shows an example of the pathway display screen when the mode 1 is selected. Fig. 26 shows an example of the pathway display screen when the mode 2 is selected.

In the display screen shown in Fig. 26, nodes regarding which the expression information are not identifiable in the expression information identification processing shown in Fig. 22 are displayed in blue, and nodes regarding which the expression data are not acquirable in the expression information acquisition processing shown in Fig. 23 are displayed in yellow. On the other hand, in the display screen shown in Fig. 25, the nodes displayed in blue and yellow in the display screen shown in Fig. 26 are not displayed.

In Fig. 26, in displaying the nodes regarding which the expression information are not identifiable in the expression information identification processing shown in Fig. 22 and the nodes regarding which the expression data are not acquirable in the expression information acquisition processing shown in Fig. 23, the information processing device 1 distinguishes these nodes by color. However, the information processing device 1 may distinguish these nodes by shape instead of color when displaying these nodes. Alternatively, the information processing device 1 may distinguish these nodes by displaying, near the nodes, a character string indicating that their expression information are not identifiable and a character string indicating that their expression data are not acquirable, instead of by changing color or shape.

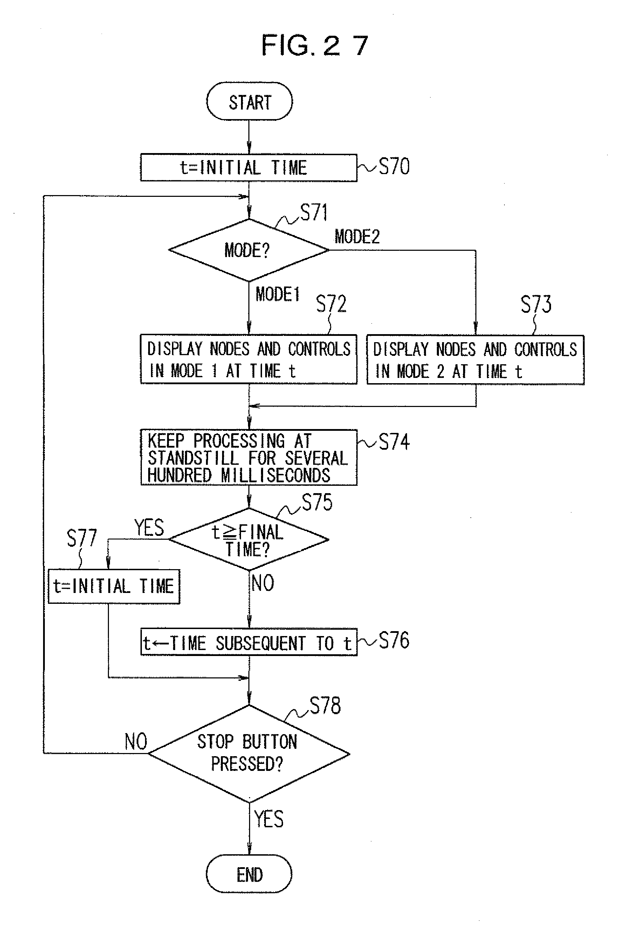

Next, an example in this embodiment of the dynamic image display processing is shown in Fig. 27. Fig. 27 is a flowchart showing an example of the dynamic image display processing. At Step S70, the dynamic image display unit 24 substitutes initial time in a variable t. At Step S71, the mode selecting unit 41 called by the dynamic image display unit 24 selects a mode based on the mode information stored in the RAM 16 or the like. Based on the selection result received from the mode selecting unit 41, the dynamic image display unit 24 goes to Step S72 when the mode 1 is set in the mode information, while going to Step S73 when the mode 2 is set in the mode information.

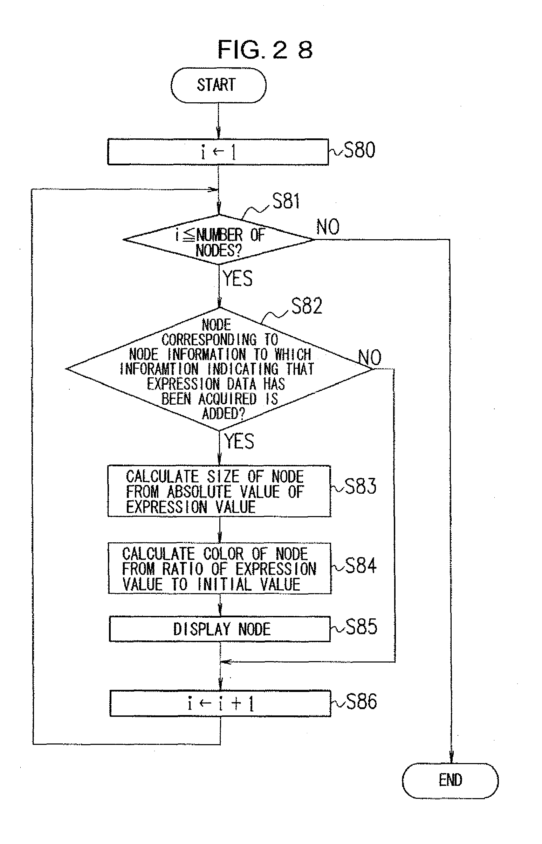

At Step S72, the dynamic image display unit 24 displays nodes, controls, and so on in the mode 1 at time t. Details of display processing at Step S72 by the dynamic image display unit 24 is shown in Fig. 28, which will be described later.

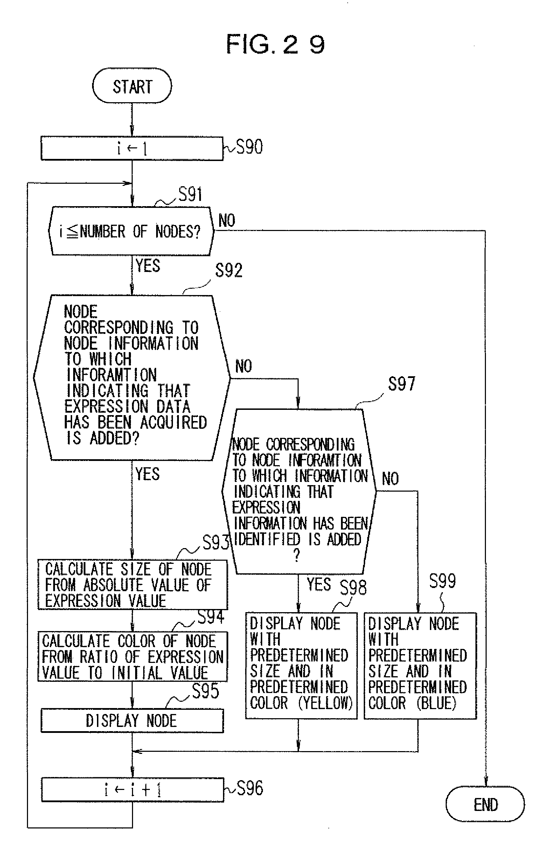

Meanwhile, at Step S73, the dynamic image display unit 24 displays nodes, controls, and so on in the mode 2 at the time t. Details of display processing at Step S73 by the dynamic image display unit 24 are shown in Fig. 29, which will be described later.

At Step S74, the dynamic image display unit 24 keeps the processing at standstill for several hundred milliseconds. After Step S74, the dynamic image display unit 24 goes to Step S75 to determine whether or not the time substituted in the variable t is equal to or more than final time which is, for example, determined in advance. The dynamic image display unit 24 goes to Step S77 when determining that the time substituted in the variable t is equal to or more than the final time which is, for example, determined in advance (YES at Step S75), while going to Step S76 when determining that the time substituted in the variable t is less than the final time which is, for example, determined in advance (NO at Step S75).

At Step S76, the dynamic image display unit 24 substitutes time subsequent to the time currently substituted in the variable t. On the other hand, at Step S77, the dynamic image display unit 24 substitutes the initial time in the variable t.

At Step S78, the dynamic image display unit 24 determines whether or not the Stop button displayed on the screen has been pressed by a user or the like. The dynamic image display unit 24 finishes the dynamic image display processing when determining that the Stop button has been pressed by the user or the like (YES at Step S78), while returning to the processing at Step S71 when determining that the Stop button has not been pressed (NO at Step S78).

Next, examples in this embodiment of the display processing of nodes and so on are shown in Fig. 28 and Fig. 29. Fig. 28 is a flowchart showing an example of the display processing of nodes and so on (No. 2).

At Step S80, the dynamic image display unit 24 substitutes 1 in a variable i. At Step S81, the dynamic image display unit 24 determines whether or not the value of the variable i is equal to or less than the number of nodes included in a pathway in a form as a display target. The dynamic image display unit 24 refers to the pathway information managed in the pathway information DB 31 or the like, and goes to Step S82 when determining that the value of the variable i is equal to or less than the number of the nodes included in the pathway in the form as a display target (YES at Step S81), while finishing the display processing when determining that the value of the variable i is more than the number of the nodes included in the pathway in the form as a display target (NO at Step S81).

At Step S82, the dynamic image display unit 24 determines whether or not the information indicating that the expression data has been acquired is added to node information regarding a node corresponding to the value of the variable i. The dynamic image display unit 24 goes to Step S83 when determining that the information indicating that the expression data has been acquired is added to the node information regarding the node corresponding to the value of the variable i (YES at Step S82), while going to Step S86 when determining that the information indicating that the expression data has been acquired is not added to the node information regarding the node corresponding to the value of the variable i (NO at Step S82).

At Step S83, the dynamic image display unit 24 calculates the size of the node to be displayed, from an absolute value of an expression value of the node corresponding to the value of the variable i. For example, as for a node included in the pathway as a display target, the dynamic image display unit 24 makes the size of the node large if an absolute value of an expression value of this node is larger than absolute values of expression values of other nodes included in the same pathway, and makes the size of the node small if the absolute value of the expression value of this node is smaller than the absolute values of the expression values of the other nodes included in the same pathway.

After Step S83, the dynamic image display unit 24 goes to Step S84 to calculate the color of the node to be displayed, from a ratio of the expression value of the node corresponding to the value of the variable i to an initial value. For example, according to the result of the ratio calculation, the dynamic image display unit 24 colors the node red if the ratio of its expression value to the initial value is large (for example, if the ratio is two times or more), colors the node black if the ratio shows no change (for example, if the ratio is about one time), and colors the node green if the ratio is small (for example, if the ratio is 0.5 times or less).

After Step S84, the dynamic image display unit 24 goes to Step S85 to display the node corresponding to the value of the variable i, according to the node size calculated at Step S83 and the node color calculated at Step S84. At Step S86, the dynamic image display unit 24 increments the value of the variable i by one and goes to Step S81.

Fig. 29 is a flowchart showing an example of the display processing of nodes and so on (No. 3). At Step S90, the dynamic image display unit 24 substitutes 1 in a variable i. At Step S91, the dynamic image display unit 24 determines whether or not the value of the variable i is equal to or less than the number of nodes included in a pathway in a form as a display target. The dynamic image display unit 24 refers to the pathway information managed in the pathway information DB 31 or the like, and goes to Step S92 when determining that the value of the variable i is equal to or less than the number of the nodes included in the pathway in the form as a display target (YES at Step S91), while finishing the display processing when determining that the value of the variable i is more than the number of the nodes included in the pathway in the form as a display target (NO at Step S91).

At Step S92, the dynamic image display unit 24 determines whether or not the information indicating that the expression data has been acquired is added to node information regarding a node corresponding to the value of the variable i. The dynamic image display unit 24 goes to Step S93 when determining that the information indicating that the expression data has been acquires is added to the node information regarding the node corresponding to the value of the variable i (YES at Step S92), while going to Step S97 when determining that the information indicating that the expression data has been acquired is not added to the node information regarding the node corresponding to the value of the variable i (NO at Step S92).

Here, processing from Step S93 to S96 is the same as the processing from Step S83 to Step S86 shown in Fig. 28, and therefore, description thereof will be omitted.

At Step S97, the dynamic image display unit 24 determines whether or not the information indicating that the expression data has been identified is added to the node information regarding the node corresponding to the value of the variable i. The dynamic image display unit 24 goes to Step S98 when determining that the information indicating that the expression information has been identified is added to the node information regarding the node corresponding to the value of the variable i (YES at Step S97), while going to Step S99 when determining that the information indicating that the expression information has been identified is not added to the node information regarding the node corresponding to the value of the variable i (NO at Step S97).

At Step S98, the dynamic image display unit 24 displays the node corresponding to the value of the variable i with size and color (for example, yellow or the like) which are, for example, determined in advance (see, for example, Fig. 26 and so on). Meanwhile, at Step S99, the dynamic image display unit 24 displays the node corresponding to the value of the variable i with size and color (for example, blue or the like) which are, for example, determined in advance (see, for example, Fig. 26 and so on).
That is, the dynamic image display unit 24 colors a node yellow if the expression information is identifiable but the expression data is not acquirable regarding this node, and colors a node blue if the expression data is not acquirable nor is the expression information identifiable regarding this node.

As described above, in displaying a node regarding which the expression information is not identifiable in the expression information identification processing shown in Fig. 22 and a node regarding which the expression data is not acquirable in the expression information acquisition processing shown in Fig. 23, the dynamic image display unit 24 can distinguish these nodes by color.
Incidentally, as previously described, the dynamic image display unit 24 may distinguish such nodes by shape instead of color. Alternatively, the dynamic image display unit 24 may distinguish such nodes by displaying, near the nodes, a character string indicating that the expression information is not identifiable and a character string indicating that the expression data is not acquirable, instead of by color or shape.

As described above, according to this embodiment, it is possible for the information processing device 1 to display or not to display a node not associated with the expression information and a node whose expression data does not exist, according to the display mode. Further, when displaying a node not associated with the expression information and a node whose expression data does not exist, the information processing device 1 displays these nodes in a distinctive manner, and therefore, a user can distinguish whether a node is associated with the expression information and whether a node is a node whose expression data does not exist.

### (Embodiment 5)

The above embodiments describe the examples where the information processing device 1 mainly operates in a stand-alone state. However, the information processing device 1 and at least one communication terminal device may be connected to each other via a network. Then, a user or the like may be capable of editing the node information, the expression information, and so on by using the communication terminal device.

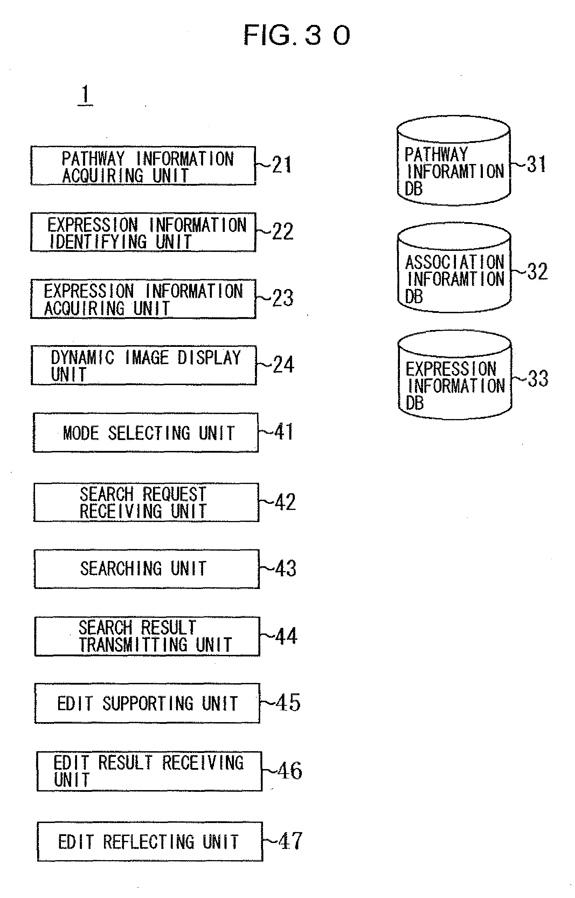

Fig. 30 is a functional block diagram of an example of the information processing device (No. 4). As shown in Fig. 30, the information processing device 1 has, as the functional configuration, a pathway information acquiring unit 21, an expression information identifying unit 22, an expression information acquiring unit 23, a dynamic image display unit 24, a mode selecting unit 41, a search request receiving unit 42, a searching unit 43, a search result transmitting unit 44, an edit supporting unit 45, an edit result receiving unit 46, an edit reflecting unit 47, a pathway information DB 31, an association information DB 32, and an expression information DB 33.

As compared with the functional configuration of the information processing device 1 shown in Fig. 24, the functional configuration of the information processing device 1 shown in Fig. 30 is different in that the search request receiving unit 42, the searching unit 43, the search result transmitting unit 44, the edit supporting unit 45, the edit result receiving unit 46, and the edit reflecting unit 47 are further included as the functional configuration.

The search request receiving unit 42 receives a search request including a search condition for information regarding a desired pathway, from the communication terminal device via the network. The searching unit 43 searches the pathway information DB 31, the association information DB 32, the expression information DB 33, and so on for the pathway-related information corresponding to the search condition included in the search request received by the search request receiving unit 42 and outputs the search result. Here, the pathway-related information refers to, for example, node information, control information, association information, expression data, and so on regarding each node included in the desired pathway.

The search result transmitting unit 44 transmits the search result outputted by the searching unit 43, to the communication terminal device as a transmitting end of the search request. The search result transmitting unit 44 further has a function of transmitting, together with the search result, an edit program capable of editing a displayed pathway such as adding and deleting a control and a node to/from the displayed pathway, when the pathway is displayed in the communication terminal device based on the pathway-related information and so on.

When receiving a request for information and so on necessary for the editing from the communication terminal device while the edit program is being executed and the edit work is underway in the communication terminal device, the edit supporting unit 45 performs processing for supporting the edit in the communication terminal device such as supplying the necessary information, in response to this request.

The edit result receiving unit 46 receives the edit result of the pathway from the communication terminal device via the network. The edit reflecting unit 47 performs processing for reflecting the edit result received by the edit result receiving unit 46, in the pathway information DB 31, the association information DB 32, the expression information DB 33, and so on. Consequently, the communication terminal device can refer to the pathway in which the edit result is reflected, by sending a search request.

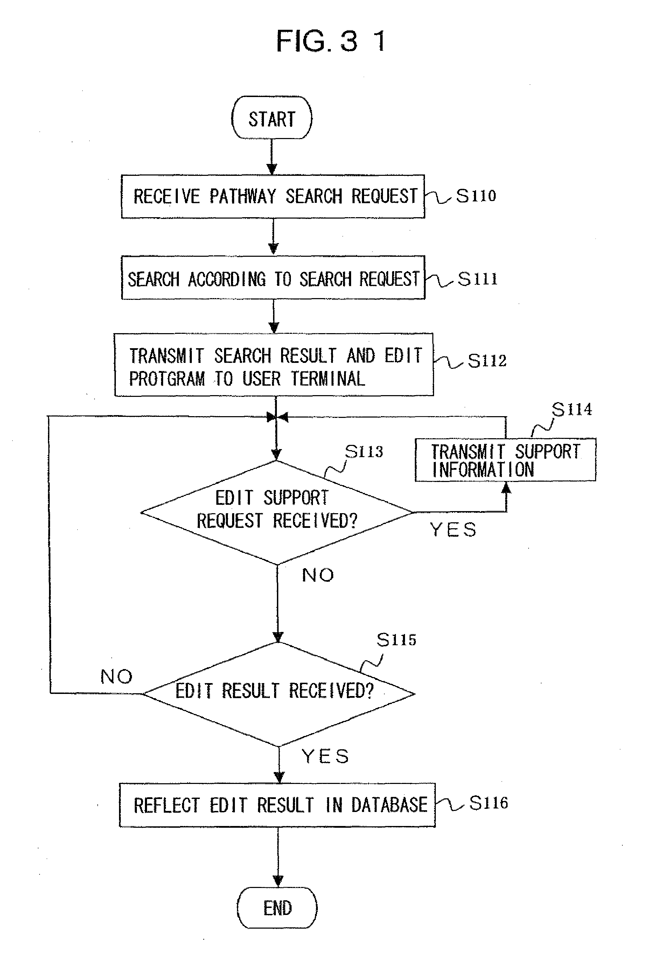

Next, an example of pathway supply processing is shown in Fig. 31. Fig. 31 is a flowchart showing an example of the pathway supply processing. As shown in Fig. 31, first at Step S110, the search request receiving unit 42 receives a search request (including a search condition) for information related to a pathway, from the communication terminal device via the network.

Next, at Step S111, the searching unit 43 searches the pathway information DB 31, the association information DB 32, the expression information DB 33, and so on for the pathway-related information corresponding to the search condition included in the search request received by the search request receiving unit 42. Next, at Step S112, the search result transmitting unit 44 transmits the search result and the edit program for editing the pathway which are outputted from the search processing unit 43, to the communication terminal device as a search requesting end. Consequently, in the communication terminal device, the pathway is displayed and an edit work to the displayed pathway can be performed. Incidentally, the edit program need not be transmitted every time after it is transmitted once to the communication terminal device, and therefore, only has to be transmitted in response to a user's request when necessary.

Next at Step S113, the edit supporting unit 45 determines whether or not an edit support request has been received from the communication terminal device. Here, the edit supporting unit 45 goes to Step S114 when determining that the edit support request has been received (YES at Step S113), and then transmits, to the communication terminal device, support information corresponding to the received edit support request. On the other hand, the flow goes to Step S115 if the edit supporting unit 45 determines at Step S113 that the edit support request has not been received (NO at S113).

At Step S115, the edit result receiving unit 46 determines whether or not the edit result has been received from the communication terminal device. Here, if the edit supporting unit 45 determines that the edit result has not been received (NO at Step S115), the flow returns to the process at Step S113. On the other hand, if the edit supporting unit 45 determines that the edit result has been received (YES at Step S115), the flow goes to Step S116.

At Step S116, the edit reflecting unit 47 performs processing for reflecting the edit result received by the edit result receiving unit 46, in the pathway information DB 31 and/or the association information DB 32 and/or the expression information DB 33.

As described above, according to this embodiment, a pathway can be easily edited and a pathway after edited can be made public.

### (Embodiment 6)

In the description of the above embodiments, the form of a control is simply a straight line, to simplify the description. However, for example, as shown in the line type ID in Fig. 6, "line" representing a control includes a plurality of types. The information processing device 1 may change the form of a control for display according to this line type ID. Incidentally, this line type ID is set by a user or the like according to an inter-node relation.

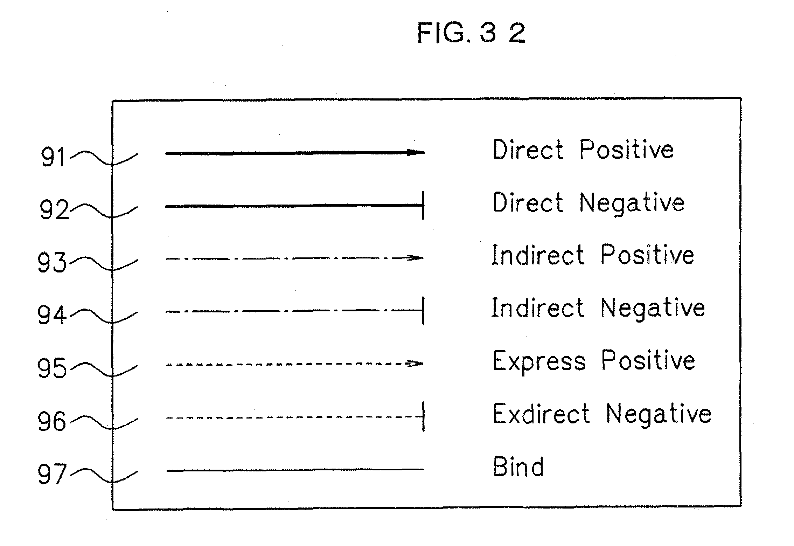

Fig. 32 is a chart showing an example of the forms of a control. Form 91 (Direct Positive) is the form of a control representing a direct and accelerative relation. Here, the direct and accelerative relation is such a relation that a node 1 modifies a node 2, a signal is transmitted from a node 1 to a node 2, a node 1 is bonded to a node 2, a node 1 changes into a node 2, etc.

Form 92 (Direct Negative) is the form of a control representing a direct and inhibitive relation. Here, the direct and inhibitive relation refers to such a relation that a node 1 inhibits a node 2, a node 1 inactivates a node 2, etc.

Form 93 (Indirect Positive) is the form of a control representing an indirect and accelerative relation. Here, the indirect and accelerative relation is such a relation that a quantitative and qualitative positive mutual relation is observed though no direct inter-node action is clear, as in a case where the activation of a node 1 increases a node 2, etc.

Form 94 (Indirect Negative) is the form of a control representing an indirect and inhibitive relation. Here, the indirect and inhibitive relation refers to such a relation that a quantitative and qualitative negative relation is observed though no direct inter-node action is clear, as in a case where the activation of a node 1 decreases a node 2, etc.

Form 95 (Express Positive) is the form representing a relation between a node inhibiting transcription and translation and a node whose expression is induced/adjusted.

Form 96 (Express Negative) is the form representing a relation between a node inhibiting transcription and translation and a node whose expression is inhibited.

Form 97 (Bind) is the form of a control representing that there is a relation.

As described above, according to this embodiment, a control can be displayed with different forms according to the inter-node relation.

### (Embodiment 7)

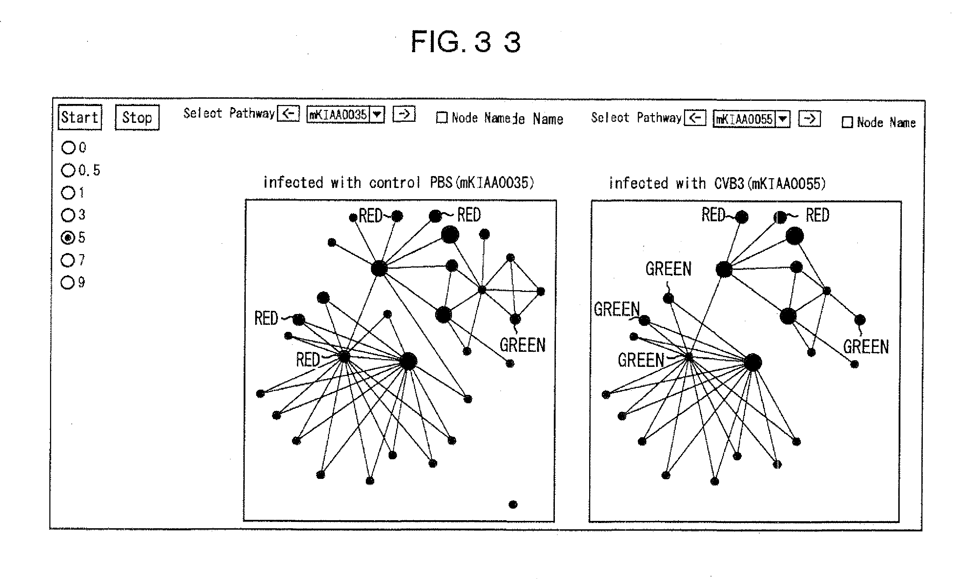

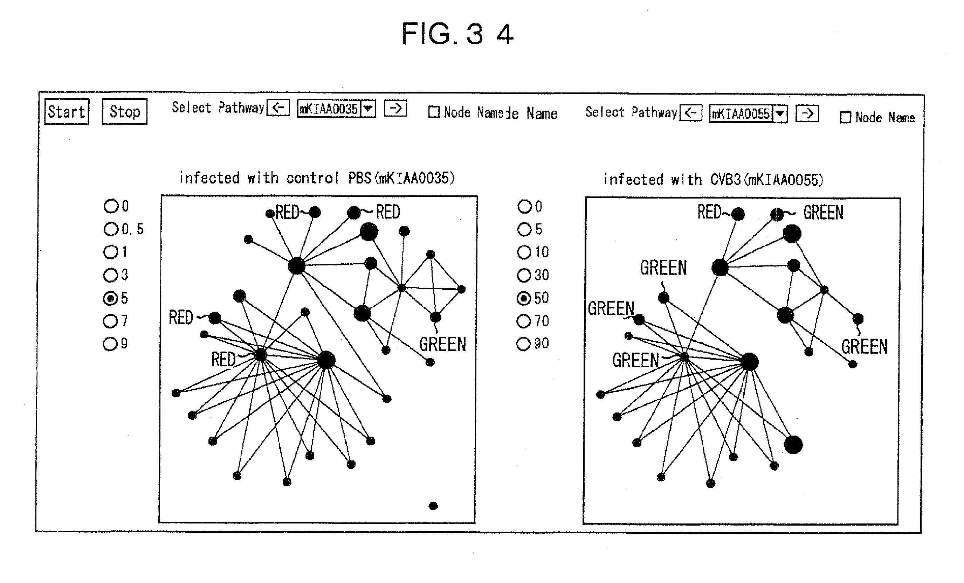

For example, the information processing device 1 may display two different pathways on one screen, display the pathways by changing them based on the same time series (see, for example, Fig. 33) or by changing them based on different time series (see, for example, Fig. 34).

Hitherto, the preferable embodiments of the present invention have been described in detail, but the present invention is not limited to such specific embodiments, and various modifications and changes can be made within the spirit of the present invention described in the claims. The above-described embodiments may be combined for implementation.

### Industrial Applicability

According to the present invention, processes of the expression of proteins, amino acids, and the like that occurs in time series can be displayed by dynamic images in the same or different time series. Further, according to the present invention, processes of the generation of metabolites that occurs in time series can be displayed by dynamic images in the same or different time series.

## Claims

1. A pathway display method in an information processing device processing information to display a pathway including: a plurality of nodes each being a symbol representing one of a protein, an amino acid, a gene, another substance associated with a gene, and a group of proteins, amino acids, genes, or other substances associated with genes; and a control representing a relation between the nodes, the method comprising:
a pathway information acquiring step in which the information processing device acquires, from a memory, pathway information regarding the pathway;
an expression information identifying step in which the information processing device identifies expression information corresponding to the pathway information acquired in said pathway information acquiring step, based on association information stored in a memory and associating the pathway information and the expression information with each other, the expression information being information regarding expression of the protein, the amino acid, the gene, the other substance associated with the gene, or the group of the proteins, the amino acids, the genes, or the other substances associated with the genes that occurs in time series;
an expression information acquiring step in which the information processing device acquires, from a memory, the expression information identified in said expression information identifying step; and
a dynamic image display step in which the information processing device displays the pathway on a screen by using the pathway information and the expression information and displays a dynamic image by changing the display of the pathway in time series.

2. The pathway display method according to claim 1, wherein in said dynamic image display step, the information processing device changes a size of each of the nodes in time series according to one of an absolute value of a value regarding the expression information and a ratio of the value regarding the expression information to an initial value.

3. The pathway display method according to claim 1, wherein in said dynamic image display step, the information processing device changes color of each of the nodes in time series according to one of an absolute value of a value regarding the expression information and a ratio of the value regarding the expression information to an initial value.

4. The pathway display method according to claim 1, wherein in said dynamic image display step, the information processing device displays a plurality of different pathways on the screen and changes the display of the pathways in time series.

5. The pathway display method according to claim 1, wherein in said dynamic image display step, the information processing device displays a plurality of the same pathways on the screen and changes the display of at least two of the plural displayed pathways based on different time series.

6. The pathway display method according to claim 1, wherein, when the expression information corresponding to the pathway information is not identifiable in said expression information identifying step, the information processing device displays, on the screen, information indicating that the expression information corresponding to the pathway information is not identifiable, in said dynamic image display step.

7. The pathway display method according to claim 1, wherein, when the expression information identified in said expression information identifying step is not acquirable from the memory in said expression information acquiring step, the information processing device displays, on the screen, information indicating that the expression information corresponding to the pathway information is not acquirable, in said dynamic image display step.

8. The pathway display method according to claim 1, further comprising a selecting step in which the information processing device selects one of at least a first display mode and a second display mode, the first display mode being a mode not to display the node for which the expression information corresponding to the pathway information is not identifiable in said expression information identifying step or for which the expression information identified in said expression information identifying step is not acquirable from the memory in said expression information acquiring step, and the second display mode being a mode to display the node as well.

9. The pathway display method according to claim 1, wherein a form of the control displayed in said dynamic image display step differs depending on the relation between the nodes represented by the control.

10. The pathway display method according to claim 1, further comprising:
an edit result receiving step in which the information processing device receives an edit result regarding the pathway from a communication terminal device with which the information processing device is capable of communicating via a network; and
a reflecting step in which the information processing device reflects the edit result received in said edit result receiving step, in the pathway information, the association information, or the expression information stored in the memory.

11. An information processing device processing information, comprising:
a pathway information acquiring unit acquiring, from a memory, pathway information regarding a pathway including: a plurality of nodes each being a symbol representing one of a protein, an amino acid, a gene, another substance associated with a gene, and a group of proteins, amino acids, genes, or other substances associated with genes; and a control representing a relation between the nodes;
an expression information identifying unit identifying expression information corresponding to the pathway information acquired in said pathway information acquiring unit, based on association information stored in a memory and associating the pathway information and the expression information with each other, the expression information being information regarding expression of the protein, the amino acid, the gene, the other substance associated with the gene, or the group of the proteins, the amino acids, the genes, or the other substances associated with the genes that occurs in time series;
an expression information acquiring unit acquiring, from a memory, the expression information identified in said expression information identifying unit; and
a dynamic image display unit displaying the pathway on a screen by using the pathway information and the expression information and displaying a dynamic image by changing the display of the pathway in time series.

12. A pathway display program product comprising program codes for causing a computer to operate as:
a pathway information acquiring unit acquiring, from a memory, pathway information regarding a pathway including: a plurality of nodes each being a symbol representing one of a protein, an amino acid, a gene, another substance associated with a gene, and a group of proteins, amino acids, genes, or other substances associated with genes; and a control representing a relation between the nodes;
an expression information identifying unit identifying expression information corresponding to the pathway information acquired in said pathway information acquiring unit, based on association information stored in a memory and associating the pathway information and the expression information with each other, the expression information being information regarding expression of the protein, the amino acid, the gene, the other substance associated with the gene, or the group of the proteins, the amino acids, the genes, or the other substances associated with the genes that occurs in time series;
an expression information acquiring unit acquiring, from a memory, the expression information identified in said expression information identifying unit; and
a dynamic image display unit displaying the pathway on a screen by using the pathway information and the expression information and displaying a dynamic image by changing the display of the pathway in time series.

13. A pathway display method in an information processing device processing information to display a pathway including nodes each representing a metabolite and a control representing a relation between the nodes, the method comprising:
a pathway information acquiring step in which the information processing device acquires, from a memory, pathway information regarding the pathway;
a generation information identifying step in which the information processing device identifies generation information corresponding to the pathway information acquired in said pathway information acquiring step, based on association information stored in a memory and associating the pathway information and the generation information with each other, the generation information being information regarding generation of the metabolite that occurs in time series;
a generation information acquiring step in which the information processing device acquires, from a memory, the generation information identified in said generation information identifying step; and
a dynamic image display step in which the information processing device displays the pathway on a screen by using the pathway information and the generation information and displays a dynamic image by changing the display of the pathway in time series.
